# EUROPEAN PATENT APPLICATION

(11) **EP 4 509 499 A1**
(43) Date of publication of application: **19.02.2025**
(21) Application number: 23787698.2
(22) Date of filing: 11.04.2023
(51) Int. Cl.: C07D 311/30, A61K 31/352, A61P 25/00, A61P 25/02, A61P 25/04, A61P 25/28, A61P 3/00, A61P 9/00, A61P 35/00

(54) **POLYMORPH OF 7,8-DIHYDROXYFLAVONE, AND PREPARATION METHOD THEREFOR**

(30) Priority: 12.04.2022 CN 202210383296
(71) Applicant: Huiankai (Xiamen) Pharmaceutical Technology Co., Ltd., Xiamen, Fujian 361028 (CN); Suzhou Glenkol Pharma Technology Co., Ltd., Suzhou, Jiangsu, 215127 (CN); Quanzhou Haichuang Pharmaceutical Technology Co., Ltd., Quanzhou, Fujian 362221 (CN)
(72) Inventor: CHEN, Jinhui, Quanzhou, Fujian 362200 (CN); HUANG, Peipei, Quanzhou, Fujian 362200 (CN)
(74) Representative: Iannone, Carlo Luigi
(86) International application number: PCT/CN2023/087608
(87) International publication number: WO 2023/198055

(57) **Abstract**

A polymorph of 7,8-dihydroxyflavone, and a preparation method therefor. The polymorph of 7,8-dihydroxyflavone has significantly improved physicochemical properties, and thus has a higher medicinal value.

## Description

### TECHNICAL FIELD

The present disclosure relates to a polymorph of 7,8-dihydroxyflavone and a preparation method therefor.

### BACKGROUND

As a special agonist to tyrosine kinase receptor B (TrkB), 7,8-dihydroxyflavone is able to activate TrkB. Numerous *in vitro* studies have demonstrated that 7,8-dihydroxyflavone has important biological roles that are mainly reflected in its good efficacy in neurodegenerative diseases including Parkinson's disease, Alzheimer's disease, depression, psychiatric disorders, post-traumatic stress disorders, autism spectrum disorders, stroke, Rett syndrome, and other diseases.

Polymorphism is a common phenomenon occurring in solid substances where the substances have two or more different crystalline structures. The polymorphic phenomenon of drugs has been an important cutting-edge topic for the research in pharmaceutical field. Different crystal structures of the same solid substance often show different physicochemical properties. As far as drugs are concerned, differences in various crystal forms are mainly reflected in stability, solubility and dissolution rate, thus affecting the efficacy of drugs and even changing their toxic side effects.

Therefore, it is of great importance to develop a crystal form of 7,8-dihydroxyflavone with excellent properties.

### SUMMARY

The present disclosure discloses a polymorph of 7,8-dihydroxyflavone and a preparation method therefor. The polymorph of 7,8-dihydroxyflavone provided by the present disclosure has more selectable pharmacoeconomic value.

In one aspect, the present disclosure provides a crystal form P of 7,8-dihydroxyflavone, which has an X-ray powder diffraction pattern comprising characteristic peaks at about 8.3 ± 0.2°, 25.0 ± 0.2°, and 25.9 ± 0.2° expressed in 2θ angles.

In an embodiment, the X-ray powder diffraction pattern further comprises characteristic peaks at about 27.4 ± 0.2° and/or 31.4 ± 0.2°.

In a further embodiment, the X-ray powder diffraction pattern further comprises characteristic peaks at about 12.5 ± 0.2°, 15.8 ± 0.2°, 18.4 ± 0.2°, 18.7 ± 0.2°, 19.4 ± 0.2° and/or 33.7 ± 0.2°.

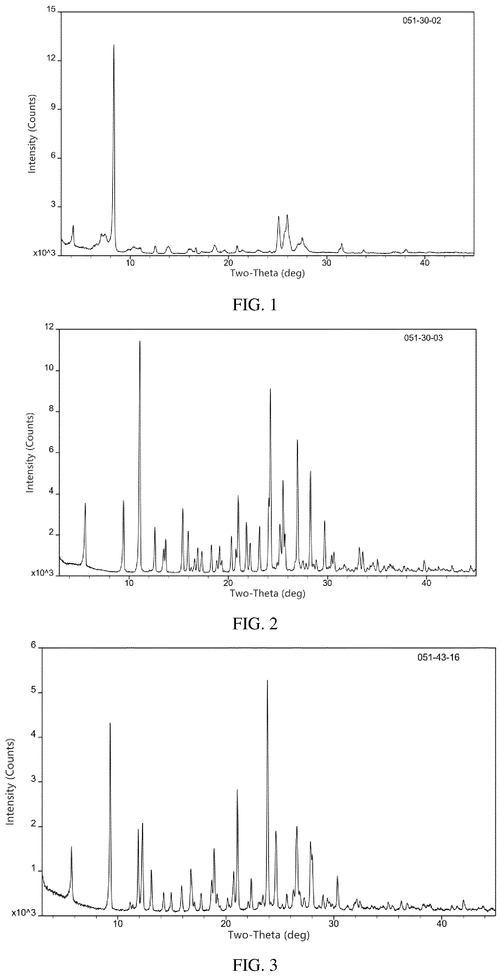

In a further embodiment, the crystal form P has an X-ray powder diffraction pattern substantially as shown in FIG. 1.

In a further embodiment, the thermogravimetric analysis curve of the crystal form P does not show obvious weight loss.

In a further embodiment, the differential scanning calorimetry curve of the crystal form P does not show heat absorption signal corresponding to weight loss.

In a further embodiment, in the dynamic vapor sorption plot of the crystal form P, the weight gain is less than 0.1% in the range of 0%-95% relative humidity, wherein "%" is the percentage of mass gain relative to the initial mass of the crystal form P of 7,8-dihydroxyflavone.

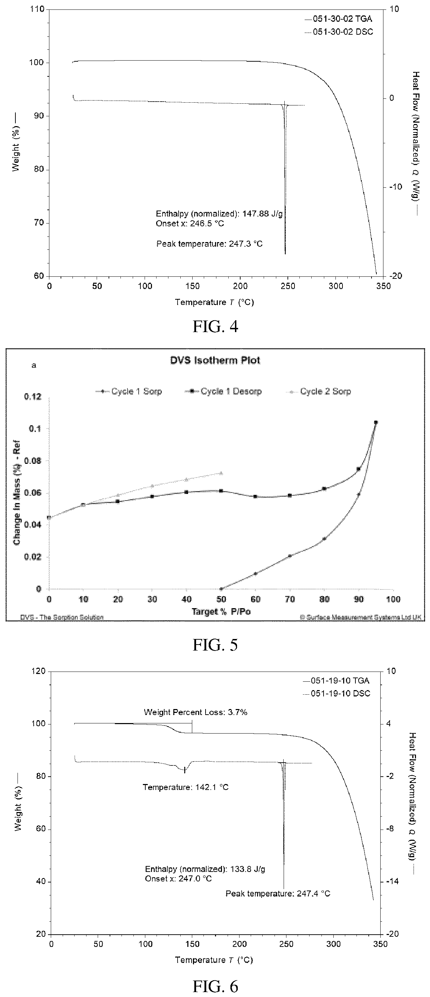

In a further embodiment, the crystal form P of 7,8-dihydroxyflavone has a thermogravimetric analysis curve and/or a differential scanning calorimetry curve substantially as shown in FIG. 4.

In a further embodiment, the crystal form P of 7,8-dihydroxyflavone has a dynamic vapor sorption graph substantially as shown in FIG. 5.

In a further embodiment, the crystal form P of 7,8-dihydroxyflavone is an anhydrous substance.

In another aspect, the present disclosure provides a crystal form C of 7,8-dihydroxyflavone, which has an X-ray powder diffraction pattern comprising characteristic peaks at about 11.1 ± 0.2°, 15.6 ± 0.2°, 27.0 ± 0.2°, and 28.3 ± 0.2° expressed in 2θ angles.

In an embodiment, the X-ray powder diffraction pattern further comprises characteristic peaks at about 5.6 ± 0.2°, 9.5 ± 0.2°, 15.5 ± 0.2°, 21.0 ± 0.2°, 24.3 ± 0.2° and/or 25.5 ± 0.2°.

In a further embodiment, the X-ray powder diffraction pattern further comprises characteristic peaks at about 12.7 ± 0.2°, 13.7 ± 0.2°, 18.3 ± 0.2°, 20.4 ± 0.2°, 21.9 ± 0.2°, 23.2 ± 0.2°, 25.2 ± 0.2° and/or 29.7 ± 0.2°.

In a further embodiment, the crystal form C has an X-ray powder diffraction pattern substantially as shown in FIG. 2.

In a further embodiment, in the thermogravimetric analysis curve of the crystal form C, the mass lost at 110°C to 150°C is about 3.7% of the mass before weight loss, wherein the "%" is a mass percentage.

In a further embodiment, the differential scanning calorimetry curve of the crystal form C shows a heat absorption signal corresponding to weight loss at about 100°C to 160°C.

**In** a further embodiment, in the dynamic vapor sorption plot of the crystal form C, the weight gain is less than 0.1% in the range of 0%-95% relative humidity, wherein "%" is the percentage of mass gain relative to the initial mass of the crystal form C of 7,8-dihydroxyflavone.

In a further embodiment, the crystal form C of 7,8-dihydroxyflavone has a thermogravimetric analysis curve and/or a differential scanning calorimetry curve substantially as shown in FIG. 6.

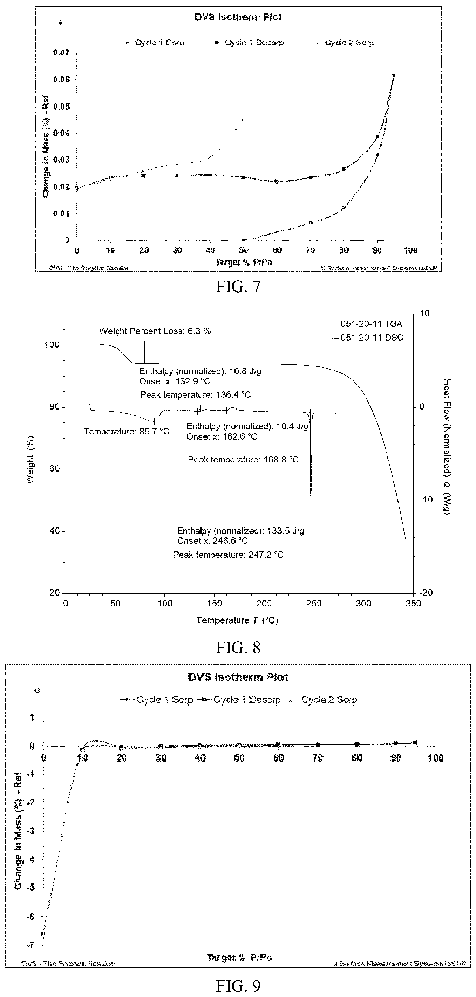

In a further embodiment, the crystal form C of 7,8-dihydroxyflavone has a dynamic vapor sorption graph substantially as shown in FIG. 7.

In a further embodiment, the crystal form C of 7,8-dihydroxyflavone is a hydrate, for example, a monohydrate.

In another aspect, the present disclosure provides a crystal form N of 7,8-dihydroxyflavone, which has an X-ray powder diffraction pattern comprising characteristic peaks at about 9.3 ± 0.2°, 23.8 ± 0.2°, 24.6 ± 0.2°, and 26.6 ± 0.2° expressed in 2θ angles.

In an embodiment, the X-ray powder diffraction pattern further comprises characteristic peaks at about 12.3 ± 0.2° and/or 21.1 ± 0.2°.

In a further embodiment, the X-ray powder diffraction pattern further comprises characteristic peaks at about 5.7 ± 0.2°, 11.9 ± 0.2°, 13.1 ± 0.2°, 14.2 ± 0.2°, 15.9 ± 0.2°, 16.8 ± 0.2°, 18.9 ± 0.2°, 20.7 ± 0.2° and/or 30.3 ± 0.2°.

In a further embodiment, the crystal form N of 7,8-dihydroxyflavone has an X-ray powder diffraction pattern substantially as shown in FIG. 3.

In a further embodiment, in the thermogravimetric analysis curve of the crystal form N, the mass lost at 40°C to 80°C is 6.3% relative to the mass before weight loss, wherein the "%" is a mass percentage.

In a further embodiment, the differential scanning calorimetry curve of the crystal form N shows an endothermic peak at about 100°C corresponding to weight loss, and exothermic signals at about 136°C and 169°C, respectively.

In a further embodiment, in the dynamic vapor sorption plot of the crystal form N, the weight gain is less than 0.1% in the range of 0%-95% relative humidity, wherein the "%" is the percentage of mass gain relative to the initial mass of the crystal form N of 7,8-dihydroxyflavone.

In a further embodiment, the crystal form N of 7,8-dihydroxyflavone has a thermogravimetric analysis curve and/or a differential scanning calorimetry curve substantially as shown in FIG. 8.

In a further embodiment, the crystal form N of 7,8-dihydroxyflavone has a dynamic vapor sorption graph substantially as shown in FIG. 9.

In a further embodiment, the crystal form N of 7,8-dihydroxyflavone is a hydrate, for example, a dihydrate.

In another aspect, the present disclosure provides a method for preparing 7,8-dihydroxyflavones in crystal forms of the present disclosure, which is one of Methods 1 to 5:
Method 1: comprising the following steps: 7,8-dihydroxyflavone is dissolved in a single solvent, and the solvent is completely volatilized to obtain a solid;
Method 2: comprising the following steps: 7,8-dihydroxyflavone is suspended in a single solvent or a binary solvent and centrifuged to obtain a solid;
Method 3: comprising the following steps: 7,8-dihydroxyflavone is dissolved in a good solvent, then the obtained solution is dropwise added into an antisolvent, and the solid is separated after precipitation;
Method 4: comprising the following steps: 7,8-dihydroxyflavone is dissolved in a good solvent, then an antisolvent is dropwise added into the obtained solution, and the solid is separated after precipitation;
Method 5: comprising the following steps: 7,8-dihydroxyflavone is suspended in an antisolvent, then added with a good solvent dropwise until the solid is dissolved, cooled to precipitate a solid, and then the solid is separated.

In an embodiment, a method for preparing a crystal form P of 7,8-dihydroxyflavone is provided, wherein in Method 2, the single solvent is n-heptane, and the binary solvent is a combination of one of 4-methyl-2-pentanone, n-propanol, N,N-dimethylformamide, ethylene glycol dimethyl ether, ethanol, and butyl formate, with one of isopropyl acetate, n-heptane, toluene, isopropanol, ethyl acetate, ethyl ether, and cyclohexane;
and/or, in Method 2, a volume ratio of the binary solvent is 1:5-1:10.

In a further embodiment, a method for preparing a crystal form P of 7,8-dihydroxyflavone is provided, wherein in Method 2, a temperature of suspension is 20°C to 60°C.

In an embodiment, 7,8-dihydroxyflavone which is prepared according to the methods of the present disclosure and has at least one feature of the crystal form P of the present disclosure is provided.

In an embodiment, a method for preparing a crystal form C of 7,8-dihydroxyflavone is provided, wherein in Method 1, the single solvent is one of isopropyl acetate, and N,N-dimethylformamide;
and/or, in Method 1, a volume-to-mass ratio of the single solvent to the 7,8-dihydroxyflavone is 5-500 mL/g;
and/or, in Method 1, the solid is precipitated by means of solvent volatilization;
and/or, in Method 2, the single solvent is water or ethyl ether; the binary solvent is selected from a combination of an organic solvent and water, a combination of butyl formate and n-heptane, a combination of ethylene glycol methyl ether and chloroform, and a combination of acetonitrile and toluene;
and/or, in Method 2, the organic solvent is selected from one or more of tetrahydrofuran, isopropanol, acetonitrile, methanol, ethanol, dimethyl sulfoxide, N,N-dimethylformamide, and ethylene glycol dimethyl ether;
and/or, in Method 2, for the binary solvent, a volume ratio of the organic solvent to the water is 1:5-1:10, a volume ratio of the butyl formate to the n-heptane is 1:1, a volume ratio of the ethylene glycol methyl ether to the chloroform is 1:5, and/or a volume ratio of the acetonitrile to the toluene is 1:1;
and/or, in Method 3, the good solvent is selected from one or more of N,N-dimethylformamide, tetrahydrofuran, and ethylene glycol dimethyl ether, and/or the antisolvent is selected from one or more of water, toluene, dichloromethane, and ethyl ether;
and/or, in Method 3, a volume ratio of the good solvent to the antisolvent is 1:1-1:10;
and/or, in Method 4, the good solvent is one or more of N,N-dimethylformamide, and isopropanol, and/or the antisolvent is one or more of chloroform, toluene, and water;
and/or, in Method 4, a volume ratio of the good solvent to the antisolvent is 1:1-1:10;
and/or, in Method 5, the antisolvent is water, and the good solvent is one or more of N,N-dimethylformamide, tetrahydrofuran, ethanol, and ethylene glycol methyl ether;
and/or, in Method 5, a volume ratio of the antisolvent to the good solvent is 1:0.8-1:1.

In a further embodiment, a method for preparing a crystal form C of 7,8-dihydroxyflavone is provided, wherein in Method 1, the single solvent is one of isopropyl acetate, and N,N-dimethylformamide;
and/or, in Method 1, the solid is precipitated at room temperature;
and/or, in Method 2, a temperature of suspension is 20°C to 60°C;
and/or, in Method 3, a temperature of dropwise addition is 15°C to 50°C;
and/or, in Method 4, a temperature of dropwise addition is 15°C to 50°C;
and/or, in Method 5, a temperature of cooling is -15°C to 4°C.

In an embodiment, 7,8-dihydroxyflavone which is prepared according to the methods of the present disclosure and has at least one feature of the crystal form C of the present disclosure is provided.

In an embodiment, a method for preparing a crystal form N of 7,8-dihydroxyflavone is provided, wherein in Method 1, the single solvent is methanol;
and/or, in Method 1, a volume-to-mass ratio of the single solvent to the 7,8-dihydroxyflavone is 5-500 mL/g;
and/or, in Method 1, the solid is precipitated by means of solvent volatilization;
and/or, in Method 2, the single solvent is methanol; the binary solvent is a combination of methanol and water;
and/or, in Method 2, a volume ratio of the methanol to the water is 1:5-1: 10;
and/or, in Method 3, the good solvent is ethanol, and the antisolvent is one or more of methyl tert-butyl ether, and toluene;
and/or, in Method 3, a volume ratio of the good solvent to the antisolvent is 1:1-1:10;
and/or, in Method 4, the good solvent is ethanol, and the antisolvent is one or more of cyclohexane, n-heptane, and water;
and/or, in Method 4, a volume ratio of the good solvent to the antisolvent is 1:1-1:10;
and/or, in Method 5, the antisolvent is one or more of toluene, isopropyl acetate, 4-methyl-2-pentanone, water, and acetonitrile, and the good solvent is one or more of ethanol, and methanol;
and/or, in Method 5, a volume ratio of the antisolvent to the good solvent is 1:0.8-1:1;

In a further embodiment, a method for preparing a crystal form N of 7,8-dihydroxyflavone is provided, wherein in Method 1, the single solvent is one of isopropyl acetate, and N,N-dimethylformamide;
and/or, in Method 1, the solid is precipitated at room temperature;
and/or, in Method 2, a temperature of suspension is 20°C to 60°C;
and/or, in Method 3, a temperature of dropwise addition is 15°C to 50°C;
and/or, in Method 4, a temperature of dropwise addition is 15°C to 50°C;
and/or, in Method 5, a temperature of cooling is -15°C to 4°C.

In an embodiment, 7,8-dihydroxyflavone which is prepared according to the methods of the present disclosure and has at least one feature of the crystal form N of the present disclosure is provided.

In another aspect, the present disclosure provides a pharmaceutical composition; and the pharmaceutical composition comprises (1) a crystal form P of 7,8-dihydroxyflavone, a crystal form C of 7,8-dihydroxyflavone, and/or a crystal form N of 7,8-dihydroxyflavone according to the present disclosure, and (2) at least one pharmaceutically acceptable excipient.

In another aspect, the present disclosure provides a method for treating a disease associated with the aberrant TrkB signaling pathway such as central nerve injury, peripheral nerve injury, neurodegenerative diseases, psychiatric diseases, hereditary neurological disorders, ophthalmological diseases, metabolic diseases, pain, cardiovascular diseases, or tumor, or other related diseases, which comprises administering to a subject (1) the crystal form P of 7,8-dihydroxyflavone, the crystal form C of 7,8-dihydroxyflavone, and/or the crystal form N of 7,8-dihydroxyflavone, and (2) at least one pharmaceutically acceptable excipient.

### BRIEF DESCRIPTION OF DRAWINGS

FIG. 1 shows an X-ray powder diffraction pattern of the crystal form P of 7,8-dihydroxyflavone obtained in Example 1.
FIG. 2 shows an X-ray powder diffraction pattern of the crystal form C of 7,8-dihydroxyflavone obtained in Example 2.
FIG. 3 shows an X-ray powder diffraction pattern of the crystal form N of 7,8-dihydroxyflavone obtained in Example 3.
FIG. 4 shows thermogravimetric analysis and differential scanning calorimetry of the crystal form P of 7,8-dihydroxyflavone obtained in Example 1.
FIG. 5 shows dynamic vapor sorption of the crystal form P of 7,8-dihydroxyflavone obtained in Example 1.
FIG. 6 shows thermogravimetric analysis and differential scanning calorimetry of the crystal form C of 7,8-dihydroxyflavone obtained in Example 2.
FIG. 7 shows dynamic vapor sorption of the crystal form C of 7,8-dihydroxyflavone obtained in Example 2.
FIG. 8 shows thermogravimetric analysis and differential scanning calorimetry of the crystal form N of 7,8-dihydroxyflavone obtained in Example 3.
FIG. 9 shows dynamic vapor sorption of the crystal form N of 7,8-dihydroxyflavone obtained in Example 3.
FIG. 10 shows X-ray powder diffraction pattern of the crystal form P of 7,8-dihydroxyflavone obtained in Example 1 before and after the dynamic vapor sorption experiment.
FIG. 11 shows polarized light microscopy analysis of the crystal form P of 7,8-dihydroxyflavone obtained in Example 1.
FIG. 12 shows a ¹H-NMR spectrum of the crystal form P of 7,8-dihydroxyflavone obtained in Example 1.
FIG. 13 shows X-ray powder diffraction pattern of the crystal form C of 7,8-dihydroxyflavone obtained in Example 2 before and after the dynamic vapor sorption experiment.
FIG. 14 shows polarized light microscopy analysis of the crystal form C of 7,8-dihydroxyflavone obtained in Example 2.
FIG. 15 shows a ¹H-NMR spectrum of the crystal form C of 7,8-dihydroxyflavone obtained in Example 2.
FIG. 16 shows X-ray powder diffraction pattern of the crystal form N of 7,8-dihydroxyflavone obtained in Example 3 before and after the dynamic vapor sorption experiment.
FIG. 17 shows polarized light microscopy analysis of the crystal form N of 7,8-dihydroxyflavone obtained in Example 3.
FIG. 18 shows a ¹H-NMR spectrum of the crystal form N of 7,8-dihydroxyflavone obtained in Example 3.
FIG. 19 shows an X-ray powder diffraction pattern of the crystal form A of 7,8-dihydroxyflavone obtained in Comparative Example 1.
FIG. 20 shows an X-ray powder diffraction pattern of the crystal form B of 7,8-dihydroxyflavone obtained in Comparative Example 2.
FIG. 21 shows an X-ray powder diffraction pattern of the crystal form E of 7,8-dihydroxyflavone obtained in Comparative Example 3.
FIG. 22 shows an X-ray powder diffraction pattern of the crystal form F of 7,8-dihydroxyflavone obtained in Comparative Example 4.
FIG. 23 shows an X-ray powder diffraction pattern of the crystal form G of 7,8-dihydroxyflavone obtained in Comparative Example 5.
FIG. 24 shows an X-ray powder diffraction pattern of the crystal form H of 7,8-dihydroxyflavone obtained in Comparative Example 6.
FIG. 25 shows an X-ray powder diffraction pattern of the crystal form I of 7,8-dihydroxyflavone obtained in Comparative Example 7.
FIG. 26 shows an X-ray powder diffraction pattern of the crystal form J of 7,8-dihydroxyflavone obtained in Comparative Example 8.
FIG. 27 shows an X-ray powder diffraction pattern of the crystal form K of 7,8-dihydroxyflavone obtained in Comparative Example 9.
FIG. 28 shows an X-ray powder diffraction pattern of the crystal form L of 7,8-dihydroxyflavone obtained in Comparative Example 10.
FIG. 29 shows an X-ray powder diffraction pattern of the crystal form M of 7,8-dihydroxyflavone obtained in Comparative Example 11.
FIG. 30 shows an X-ray powder diffraction pattern of the crystal form O of 7,8-dihydroxyflavone obtained in Comparative Example 12.
FIG. 31 shows an X-ray powder diffraction pattern of the crystal form Q of 7,8-dihydroxyflavone obtained in Comparative Example 13.
FIG. 32 shows an X-ray powder diffraction pattern of the crystal form R of 7,8-dihydroxyflavone obtained in Comparative Example 14.
FIG. 33 shows an X-ray powder diffraction pattern of the crystal form S of 7,8-dihydroxyflavone obtained in Comparative Example 15.
FIG. 34 shows an X-ray powder diffraction pattern of the crystal form T of 7,8-dihydroxyflavone obtained in Comparative Example 16.
FIG. 35 shows an X-ray powder diffraction pattern of the crystal form P of 7,8-dihydroxyflavone obtained in Performance Example 1.
FIG. 36 shows an X-ray powder diffraction pattern of the crystal form C of 7,8-dihydroxyflavone obtained in Performance Example 1.
FIG. 37 shows an X-ray powder diffraction pattern of the crystal form N of 7,8-dihydroxyflavone obtained in Performance Example 1.
FIG. 38 shows an X-ray powder diffraction pattern of the crystal form P of 7,8-dihydroxyflavone obtained in Performance Example 2.
FIG. 39 shows an X-ray powder diffraction pattern of the crystal form C of 7,8-dihydroxyflavone obtained in Performance Example 2.
FIG. 40 shows an X-ray powder diffraction pattern of the crystal form N of 7,8-dihydroxyflavone obtained in Performance Example 2.

### DETAILED DESCRIPTION

In the present disclosure, the 7,8-dihydroxyflavone has a formula as follows:

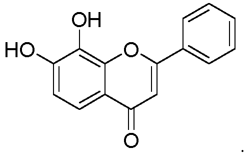

Unless explicitly specified, the term 7,8-dihydroxyflavone as used in the present disclosure does not necessarily have any particular physical states, which may be amorphous or in any crystal forms.

**In** an embodiment, the present disclosure provides a 7,8-dihydroxyflavone at least having crystal form P. The crystal form P is characterized by an X-ray diffraction pattern thereof, which is characterized by X-ray diffraction peaks at 2θ values of about 8.3 ± 0.2°, 25.0 ± 0.2°, and 25.9 ± 0.2°. In a further embodiment, the X-ray diffraction pattern of crystal form P also has X-ray diffraction peaks at 2θ values of about 27.4 ± 0.2° and/or 31.4 ± 0.2°. In a further embodiment, the crystal form P is an anhydrous substance. In a further embodiment, the X-ray diffraction pattern of crystal form P is shown in FIG. 1. In a further embodiment, the positions of the characteristic X-ray diffraction peaks of crystal form P are shown in Table 1 along with the positions of the characteristic X-ray diffraction peaks of the other crystal forms of 7,8-dihydroxyflavone.

In one performance embodiment, the present disclosure relates to a crystal form P of 7,8-dihydroxyflavone which is stable and does not suffer from crystal transformation.

In an embodiment, the present disclosure provides a 7,8-dihydroxyflavone at least having crystal form C. The crystal form C is characterized by an X-ray diffraction pattern thereof, which is characterized by X-ray diffraction peaks at 2θ values of about 5.6 ± 0.2°, 9.5 ± 0.2°, 11.1 ± 0.2°, 15.5 ± 0.2°, 15.6 ± 0.2°, 21.0 ± 0.2°, 24.3 ± 0.2°, 25.5 ± 0.2°, 27.0 ± 0.2°, and 28.3 ± 0.2°. In a further embodiment, the crystal form C is a hydrate, for example, a monohydrate. In a further embodiment, the X-ray diffraction pattern of crystal form C is shown in FIG. 2. In a further embodiment, the positions of the characteristic X-ray diffraction peaks of crystal form C are shown in Table 1 along with the positions of the characteristic X-ray diffraction peaks of the other crystal forms of 7,8-dihydroxyflavone.

In one performance embodiment, the present disclosure relates to a crystal form C of 7,8-dihydroxyflavone which is stable and does not suffer from crystal transformation.

In an embodiment, the present disclosure provides a 7,8-dihydroxyflavone at least having crystal form N. The crystal form N is characterized by an X-ray diffraction pattern thereof, which is characterized by X-ray diffraction peaks at 2θ values of about 9.3 ± 0.2°, 23.8 ± 0.2°, 24.6 ± 0.2°, and 26.6 ± 0.2°. In a further embodiment, the X-ray diffraction pattern of crystal form N also has X-ray diffraction peaks at 2θ values of about 12.3 ± 0.2° and/or 21.1 ± 0.2°. In a further embodiment, the crystal form N is a hydrate, for example, a dihydrate. In a further embodiment, the X-ray diffraction pattern of crystal form N is shown in FIG. 3. In a further embodiment, the positions of the characteristic X-ray diffraction peaks of crystal form N are shown in Table 1 along with the positions of the characteristic X-ray diffraction peaks of the other crystal forms of 7,8-dihydroxyflavone.

In one performance embodiment, the present disclosure relates to a crystal form N of 7,8-dihydroxyflavone which is stable and does not suffer from crystal transformation.

The present disclosure relates to methods for preparing 7,8-dihydroxyflavones in crystal forms comprising Methods 1 to 5:
Method 1: comprising the following steps: 7,8-dihydroxyflavone is dissolved in a single solvent, and the solvent is completely volatilized to obtain a solid;
Method 2: comprising the following steps: 7,8-dihydroxyflavone is suspended in a single solvent or a binary solvent and centrifuged to obtain a solid;
Method 3: comprising the following steps: 7,8-dihydroxyflavone is dissolved in a good solvent, then added dropwise to an antisolvent, and centrifuged after solid precipitation to obtain a solid;
Method 4: comprising the following steps: 7,8-dihydroxyflavone is dissolved in a good solvent, then added dropwise to an antisolvent, and centrifuged after solid precipitation to obtain a solid;
Method 5: comprising the following steps: 7,8-dihydroxyflavone is suspended in an antisolvent, then added with a good solvent dropwise until the solid is dissolved, cooled to precipitate a solid and then centrifuged to obtain the solid.

In an embodiment, the present disclosure relates to a method for preparing a 7,8-dihydroxyflavone at least having crystal form P, in particular a 7,8-dihydroxyflavone of pure crystal form P. The method may be, for example:
in Method 2, the single solvent is n-heptane, and the binary solvent is a combination of one of 4-methyl-2-pentanone, n-propanol, N,N-dimethylformamide, ethylene glycol dimethyl ether, ethanol, and butyl formate, with one of isopropyl acetate, n-heptane, toluene, isopropanol, ethyl acetate, ethyl ether, and cyclohexane; and further preferably, the single solvent is n-heptane, and the binary solvent is a combination of ethanol and ethyl acetate;
and/or, in Method 2, a volume ratio of the binary solvent is 1:5-1:10; further preferably, the volume ratio of the binary solvent is 1:10;
in Method 2, the suspension is performed at a temperature of 20°C to 60°C; further preferably, the suspension is performed at a temperature of 50°C.

In an embodiment, the present disclosure relates to a method for preparing a 7,8-dihydroxyflavone at least having crystal form C, in particular a 7,8-dihydroxyflavone of pure crystal form C. The method may be, for example:
in Method 1, the single solvent is one of isopropyl acetate, and N,N-dimethylformamide; further preferably, the single solvent is N,N-dimethylformamide;
and/or, in Method 1, a volume-to-mass ratio of the single solvent to the 7,8-dihydroxyflavone is 5-500 mL/g; further preferably, the volume-to-mass ratio of the single solvent to the 7,8-dihydroxyflavone is 5 mL/g;
and/or, in Method 1, the solid precipitation is performed by means of solvent volatilization;
and/or, in Method 2, the single solvent is water, or ethyl ether; further preferably, the single solvent is water; the binary solvent is a combination of an organic solvent and water, a combination of butyl formate and n-heptane, a combination of ethylene glycol methyl ether and chloroform, and a combination of acetonitrile and toluene; further preferably, the binary solvent is a combination of an organic solvent and water;
and/or, in Method 2, the organic solvent is one of tetrahydrofuran, isopropanol, acetonitrile, methanol, ethanol, dimethyl sulfoxide, N,N-dimethylformamide, and ethylene glycol dimethyl ether; further preferably the organic solvent is tetrahydrofuran;
and/or, in Method 2, for the binary solvent, a volume ratio of the organic solvent to the water is 1:5-1:10, a volume ratio of the butyl formate to the n-heptane is 1:1, a volume ratio of the ethylene glycol methyl ether to the chloroform is 1:5, and a volume ratio of the acetonitrile to the toluene is 1:1; further preferably, for the binary solvent, a volume ratio of the organic solvent to the water is 1:5;
and/or, in Method 3, the good solvent is one of N,N-dimethylformamide, tetrahydrofuran, and ethylene glycol dimethyl ether, and the antisolvent is one of water, toluene, dichloromethane, and ethyl ether; further preferably, the good solvent is N,N-dimethylformamide and the antisolvent is water;
and/or, in Method 3, a volume ratio of the good solvent to the antisolvent is 1:1-1:10; further preferably, the volume ratio of the good solvent to the antisolvent is 1:1;
and/or, in Method 4, the good solvent is one of N,N-dimethylformamide, and isopropanol, and the antisolvent is one of chloroform, toluene, and water; further preferably, the good solvent is N,N-dimethylformamide and the antisolvent is chloroform;
and/or, in Method 4, a volume ratio of the good solvent to the antisolvent is 1:1-1:10; further preferably, the volume ratio of the good solvent to the antisolvent is 1:1;
and/or, in Method 5, the antisolvent is water, and the good solvent is one of N,N-dimethylformamide, tetrahydrofuran, ethanol, and ethylene glycol methyl ether; further preferably, the antisolvent is water and the good solvent is N,N-dimethylformamide;
and/or, in Method 5, a volume ratio of the antisolvent to the good solvent is 1:0.8-1:1; further preferably, the volume ratio of the antisolvent to the good solvent is 1:0.8;
in Method 1, the single solvent is one of isopropyl acetate, and N,N-dimethylformamide; further preferably, the single solvent is N,N-dimethylformamide;
and/or, in Method 1, the solid precipitation is performed at room temperature;
and/or, in Method 2, the suspension is performed at a temperature of 20°C to 60°C; further preferably, the suspension is performed at a temperature of 25°C;
and/or, in Method 3, the dropwise addition is performed at a temperature of 15°C to 50°C; further preferably, the dropwise addition is performed at a temperature of 25°C;
and/or, in Method 4, the dropwise addition is performed at a temperature of 15°C to 50°C; further preferably, the dropwise addition is performed at a temperature of 25°C;
and/or, in Method 5, the cooling is performed at a temperature of -15°C to 4°C.

The present disclosure relates to a method for preparing a 7,8-dihydroxyflavone at least having crystal form N, in particular a 7,8-dihydroxyflavone of pure crystal form N. The method may be, for example:
in Method 1, the single solvent is methanol;
and/or, in Method 1, a volume-to-mass ratio of the single solvent to the 7,8-dihydroxyflavone is 5-500 mL/g; further preferably, the volume-to-mass ratio of the single solvent to the 7,8-dihydroxyflavone is 5 mL/g
and/or, in Method 1, the solid precipitation is performed by means of solvent volatilization;
and/or, in Method 2, the single solvent is methanol; the binary solvent is a combination of methanol and water;
and/or, in Method 2, for the binary solvent, a volume ratio of the methanol to the water is 1:5-1:10; further preferably, for the binary solvent, the volume ratio of the methanol to the water is 1:5;
and/or, in Method 3, the good solvent is ethanol, and the antisolvent is one of methyl tert-butyl ether, and toluene; further preferably, the antisolvent is methyl tert-butyl ether;
and/or, in Method 3, a volume ratio of the good solvent to the antisolvent is 1:1-1:10; further preferably, a volume ratio of the good solvent to the antisolvent is 1:1;
and/or, in Method 4, the good solvent is ethanol, and the antisolvent is one of cyclohexane, n-heptane, and water; further preferably, the antisolvent is cyclohexane;
and/or, in Method 4, a volume ratio of the good solvent to the antisolvent is 1:1-1:10; further preferably, the volume ratio of the good solvent to the antisolvent is 1:1;
and/or, in Method 5, the antisolvent is one of toluene, isopropyl acetate, 4-methyl-2-pentanone, water, and acetonitrile, and the good solvent is one of ethanol, and methanol; further preferably, the antisolvent is toluene and the good solvent is ethanol;
and/or, in Method 5, a volume ratio of the antisolvent to the good solvent is 1:0.8-1:1; further preferably, the volume ratio of the antisolvent to the good solvent is 1:0.8;
and/or, in Method 1, the single solvent is methanol;
and/or, in Method 1, the precipitated solid is performed at room temperature;
and/or, in Method 2, the suspension is performed at a temperature of 20°C to 60°C; further preferably, the suspension is performed at a temperature of 50°C;
and/or, in Method 3, the dropwise addition is performed at a temperature of 15°C to 50°C; further preferably, the dropwise addition is performed at a temperature of 25°C;
and/or, in Method 4, the dropwise addition is performed at a temperature of 15°C to 50°C; further preferably, the dropwise addition is performed at a temperature of 25°C;
and/or, in Method 5, the cooling is performed at a temperature of -15°C to 4°C; further preferably, the cooling is performed at a temperature of 0°C.

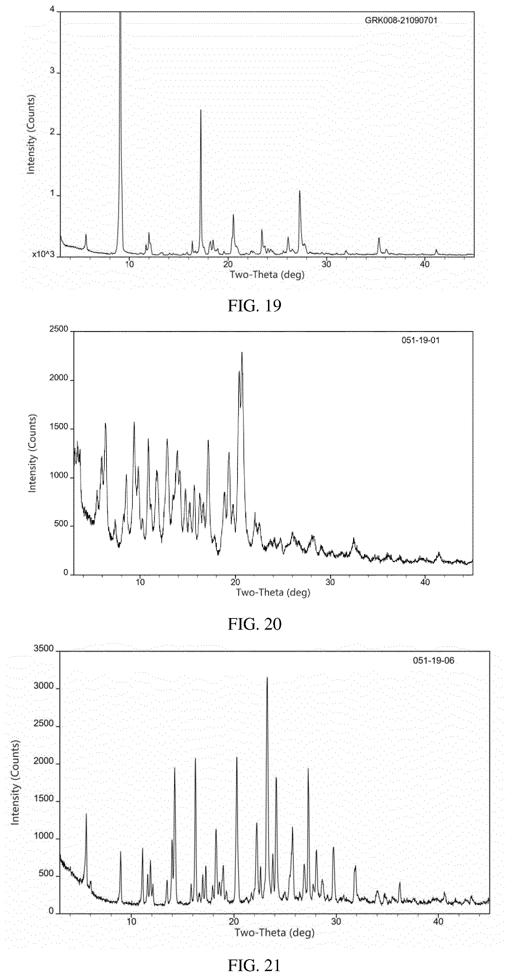

In a comparative embodiment, the present disclosure provides a 7,8-dihydroxyflavone at least having crystal form A. The crystal form A is characterized by an X-ray diffraction pattern thereof, which is characterized by X-ray diffraction peaks at 2θ values of about 9.1 ± 0.2°, 17.2 ± 0.2°, 20.5 ± 0.2°, and 27.3 ± 0.2°; the crystal form A is a hydrate. The X-ray diffraction pattern of the crystal form A is shown in FIG. 19. The positions of the characteristic X-ray diffraction peaks of the crystal form A are shown in Table 1 along with the positions of the characteristic X-ray diffraction peaks of the other crystal forms of 7,8-dihydroxyflavone.

On one hand, the crystal form A will convert to the crystal form N under sufficient drying, thus cannot exist for a long period undergo drying conditions; on the other hand, the crystal form A will convert to mixed crystals containing the crystal form F when heated to 130°C, and when heated to 160°C, the crystal form A will convert to mixed crystals containing the crystal form F and the crystal form J.

In a comparative embodiment, the present disclosure provides a 7,8-dihydroxyflavone at least having crystal form B. The crystal form B is characterized by an X-ray diffraction pattern thereof, which is characterized by X-ray diffraction peaks at 2θ values of about 6.4 ± 0.2°, 9.4 ± 0.2°, 10.9 ± 0.2°, 12.8 ± 0.2°, 13.9 ± 0.2°, 17.2 ± 0.2°, 18.8 ± 0.2°, 20.4 ± 0.2°, and 20.7 ± 0.2°. The crystal form B is an anhydrous substance containing a little absorbed solvent. The X-ray diffraction pattern of the crystal form B is shown in FIG. 20. The positions of the characteristic X-ray diffraction peaks of the crystal form B are shown in Table 1 along with the positions of the characteristic X-ray diffraction peaks of the other crystal forms of 7,8-dihydroxyflavone.

On one hand, the crystal form B will tend to convert to the crystal form C under room temperature conditions, thus cannot exist at room temperature for a long period; on the other hand, some characteristic peaks of the crystal form J will appear when the crystal form B is heated to 110°C, and the crystal form B will convert to mixed crystals containing the crystal form F and the crystal form J at 180°C.

In a comparative embodiment, the present disclosure provides a 7,8-dihydroxyflavone at least having crystal form E. The crystal form E is characterized by an X-ray diffraction pattern thereof, which is characterized by X-ray diffraction peaks at 2θ values of about 14.2 ± 0.2°, 16.3 ± 0.2°, 20.3 ± 0.2°, 23.3 ± 0.2°, 24.2 ± 0.2°, and 27.3 ± 0.2°. The crystal form E is a hydrate or a dimethyl sulfoxide solvate. The X-ray diffraction pattern of the crystal form E is shown in FIG. 21. The positions of the characteristic X-ray diffraction peaks of the crystal form E are shown in Table 1 along with the positions of the characteristic X-ray diffraction peaks of the other crystal forms of 7,8-dihydroxyflavone.

The crystal form E will convert to the crystal form N at room temperature.

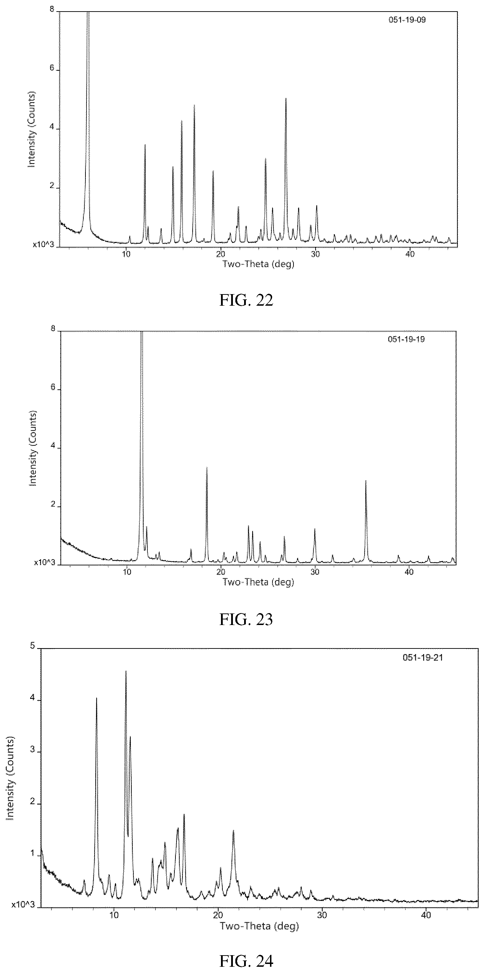

In a comparative embodiment, the present disclosure provides a 7,8-dihydroxyflavone at least having crystal form F. The crystal form F is characterized by an X-ray diffraction pattern thereof, which is characterized by X-ray diffraction peaks at 2θ values of about 6.0 ± 0.2°, 15.9 ± 0.2°, 17.2 ± 0.2°, 19.2 ± 0.2°, and 26.9 ± 0.2°. The crystal form F is an anhydrous substance containing a little absorbed solvent. The X-ray diffraction pattern of the crystal form F is shown in FIG. 22. The positions of the characteristic X-ray diffraction peaks of the crystal form F are shown in Table 1 along with the positions of the characteristic X-ray diffraction peaks of the other crystal forms of 7,8-dihydroxyflavone.

On one hand, the crystal form F will convert to the crystal form B at room temperature, thus cannot exist at room temperature for a long period; on the other hand, the crystal form F will convert to mixed crystals of the crystal form J and the crystal form P when heated to 220°C.

In a comparative embodiment, the present disclosure provides a 7,8-dihydroxyflavone at least having crystal form G. The crystal form G is characterized by an X-ray diffraction pattern thereof, which is characterized by X-ray diffraction peaks at 2θ values of about 11.6 ± 0.2°, 18.5 ± 0.2°, and 35.4 ± 0.2°. The crystal form G is a hydrate. The X-ray diffraction pattern of the crystal form G is shown in FIG. 23. The positions of the characteristic X-ray diffraction peaks of the crystal form G are shown in Table 1 along with the positions of the characteristic X-ray diffraction peaks of the other crystal forms of 7,8-dihydroxyflavone.

The crystal form G will convert to the crystal form T which has a small amount of crystal form J when heated to 140°C.

In a comparative embodiment, the present disclosure provides a 7,8-dihydroxyflavone at least having crystal form H. The crystal form H is characterized by an X-ray diffraction pattern thereof, which is characterized by X-ray diffraction peaks at 2θ values of about 8.3 ± 0.2°, 11.2 ± 0.2°, 11.6 ± 0.2°, 16.7 ± 0.2°, and 21.5 ± 0.2°. The X-ray diffraction pattern of the crystal form H is shown in FIG. 24. The positions of the characteristic X-ray diffraction peaks of the crystal form H are shown in Table 1 along with the positions of the characteristic X-ray diffraction peaks of the other crystal forms of 7,8-dihydroxyflavone.

The crystal form H will convert to other crystal forms at room temperature and cannot exist at room temperature for a long period.

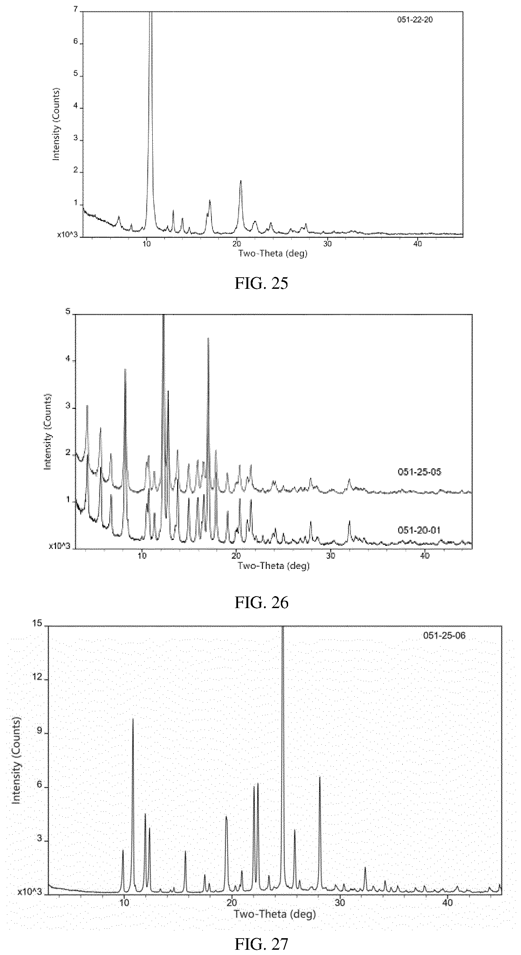

In a comparative embodiment, the present disclosure provides a 7,8-dihydroxyflavone at least having crystal form I. The crystal form I is characterized by an X-ray diffraction pattern thereof, which is characterized by X-ray diffraction peaks at 2θ values of about 10.5 ± 0.2°, 17.0 ± 0.2°, and 20.5 ± 0.2°. The X-ray diffraction pattern of the crystal form I is shown in FIG. 25. The positions of the characteristic X-ray diffraction peaks of the crystal form I are shown in Table 1 along with the positions of the characteristic X-ray diffraction peaks of the other crystal forms of 7,8-dihydroxyflavone.

In a comparative embodiment, the present disclosure provides a 7,8-dihydroxyflavone at least having crystal form J. The crystal form J is characterized by an X-ray diffraction pattern thereof, which is characterized by X-ray diffraction peaks at 2θ values of about 4.3 ± 0.2°, 5.7 ± 0.2°, 6.8 ± 0.2°, 8.3 ± 0.2°, 12.4 ± 0.2°, 12.8 ± 0.2°, 17.2 ± 0.2°, and 18.0 ± 0.2°. The crystal form J is an anhydrous substance. The X-ray diffraction pattern of the crystal form J is shown in FIG. 26. The positions of the characteristic X-ray diffraction peaks of the crystal form J are shown in Table 1 along with the positions of the characteristic X-ray diffraction peaks of the other crystal forms of 7,8-dihydroxyflavone.

In a comparative embodiment, the present disclosure provides a 7,8-dihydroxyflavone at least having crystal form K. The crystal form K is characterized by an X-ray diffraction pattern thereof, which is characterized by X-ray diffraction peaks at 2θ values of about 10.8 ± 0.2°, 19.5 ± 0.2°, 22.1 ± 0.2°, 22.4 ± 0.2°, 24.7 ± 0.2°, and 28.2 ± 0.2°. The crystal form K is a dioxane solvate. The X-ray diffraction pattern of the crystal form K is shown in FIG. 27. The positions of the characteristic X-ray diffraction peaks of the crystal form K are shown in Table 1 along with the positions of the characteristic X-ray diffraction peaks of the other crystal forms of 7,8-dihydroxyflavone.

The crystal form K will convert to mixed crystals of the crystal form F and the crystal form J when heated to 150°C and then cooled to room temperature.

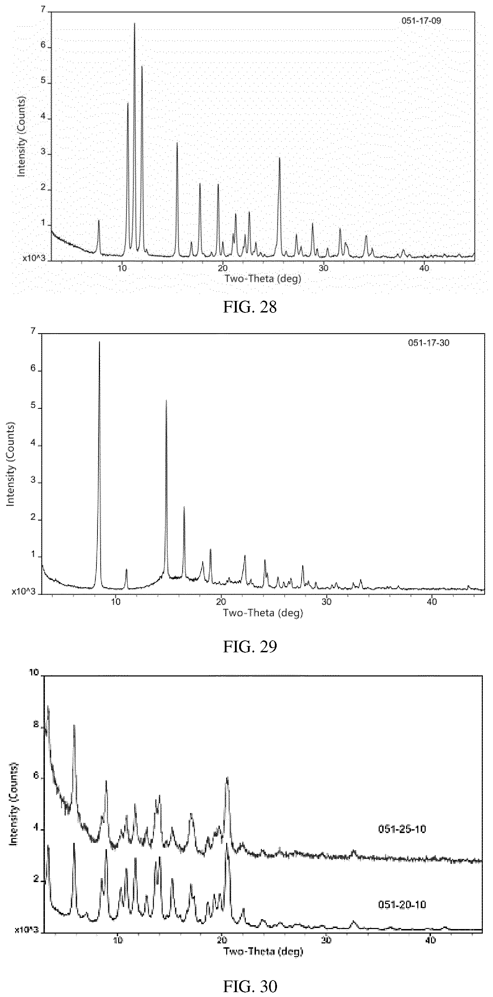

In a comparative embodiment, the present disclosure provides a 7,8-dihydroxyflavone at least having crystal form L. The crystal form L is characterized by an X-ray diffraction pattern thereof, which is characterized by X-ray diffraction peaks at 2θ values of about 10.6 ± 0.2°, 11.3 ± 0.2°, 12.0 ± 0.2°, 15.5 ± 0.2°, 17.7 ± 0.2°, 19.5 ± 0.2°, and 22.6 ± 0.2°. The X-ray diffraction pattern of the crystal form L is shown in FIG. 28. The positions of the characteristic X-ray diffraction peaks of the crystal form L are shown in Table 1 along with the positions of the characteristic X-ray diffraction peaks of the other crystal forms of 7,8-dihydroxyflavone.

In a comparative embodiment, the present disclosure provides a 7,8-dihydroxyflavone at least having crystal form M. The crystal form M is characterized by an X-ray diffraction pattern thereof, which is characterized by X-ray diffraction peaks at 2θ values of about 8.5 ± 0.2°, 14.8 ± 0.2°, 16.5 ± 0.2°, and 19.0 ± 0.2°. The crystal form M is an acetone solvate. The X-ray diffraction pattern of the crystal form M is shown in FIG. 29. The positions of the characteristic X-ray diffraction peaks of the crystal form M are shown in Table 1 along with the positions of the characteristic X-ray diffraction peaks of the other crystal forms of 7,8-dihydroxyflavone.

The crystal form M will convert to the crystal form C at room temperature and cannot exist at room temperature for a long period.

In a comparative embodiment, the present disclosure provides a 7,8-dihydroxyflavone at least having crystal form O. The crystal form O is characterized by an X-ray diffraction pattern thereof, which is characterized by X-ray diffraction peaks at 2θ values of about 3.4 ± 0.2°, 5.9 ± 0.2°, 8.9 ± 0.2°, 10.9 ± 0.2°, 11.7 ± 0.2°, 13.6 ± 0.2°, 14.1 ± 0.2°, 15.2 ± 0.2°, 20.4 ± 0.2°, and 20.7 ± 0.2°. The crystal form O is an anhydrous substance having a little solvent residue or a chloroform solvate. The X-ray diffraction pattern of the crystal form O is shown in FIG. 30. The positions of the characteristic X-ray diffraction peaks of the crystal form O are shown in Table 1 along with the positions of the characteristic X-ray diffraction peaks of the other crystal forms of 7,8-dihydroxyflavone.

The crystal form O will convert to the crystal form C at room temperature and cannot exist at room temperature for a long period.

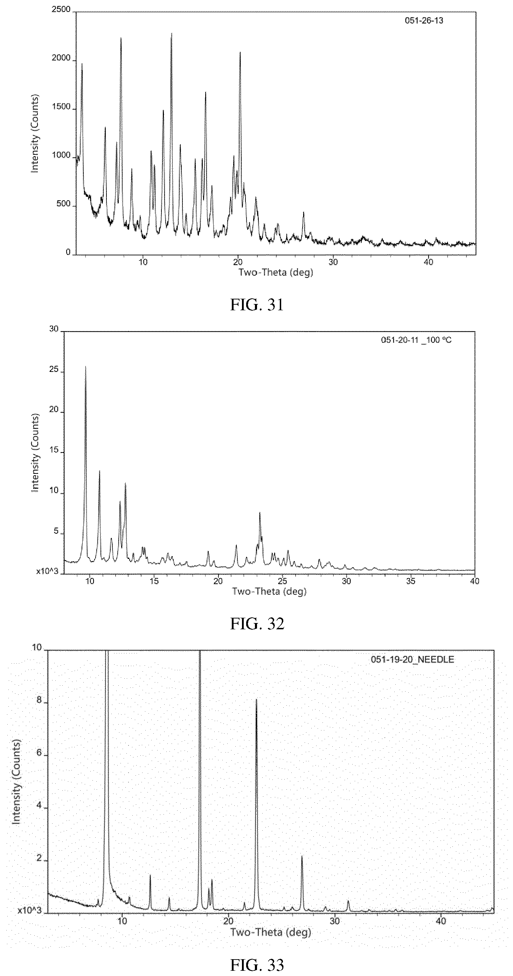

In a comparative embodiment, the present disclosure provides a 7,8-dihydroxyflavone at least having crystal form Q. The crystal form Q is characterized by an X-ray diffraction pattern thereof, which is characterized by X-ray diffraction peaks at 2θ values of about 3.6 ± 0.2°, 7.7 ± 0.2°, 12.1 ± 0.2°, 13.0 ± 0.2°, 16.6 ± 0.2°, and 20.2 ± 0.2°. The X-ray diffraction pattern of the crystal form Q is shown in FIG. 31. The positions of the characteristic X-ray diffraction peaks of the crystal form Q are shown in Table 1 along with the positions of the characteristic X-ray diffraction peaks of the other crystal forms of 7,8-dihydroxyflavone.

The crystal form Q will convert to the crystal form C at room temperature and cannot exist at room temperature for a long period.

In a comparative embodiment, the present disclosure provides a 7,8-dihydroxyflavone at least having crystal form R. The crystal form R is characterized by an X-ray diffraction pattern thereof, which is characterized by X-ray diffraction peaks at 2θ values. The X-ray diffraction pattern of the crystal form R is shown in FIG. 32. The positions of the characteristic X-ray diffraction peaks of the crystal form R are shown in Table 1 along with the positions of the characteristic X-ray diffraction peaks of the other crystal forms of 7,8-dihydroxyflavone.

The crystal form R can only be obtained by heating the crystal form N to 100°C and cannot exist at room temperature for a long period.

In a comparative embodiment, the present disclosure provides a 7,8-dihydroxyflavone at least having crystal form S. The crystal form S is characterized by an X-ray diffraction pattern thereof, which is characterized by X-ray diffraction peaks at 2θ values of about 8.6 ± 0.2°, 17.3 ± 0.2°, and 22.6 ± 0.2°. The X-ray diffraction pattern of the crystal form S is shown in FIG. 33. The positions of the characteristic X-ray diffraction peaks of the crystal form S are shown in Table 1 along with the positions of the characteristic X-ray diffraction peaks of the other crystal forms of 7,8-dihydroxyflavone.

The crystal form S will convert to the crystal form C at room temperature and cannot exist at room temperature for a long period.

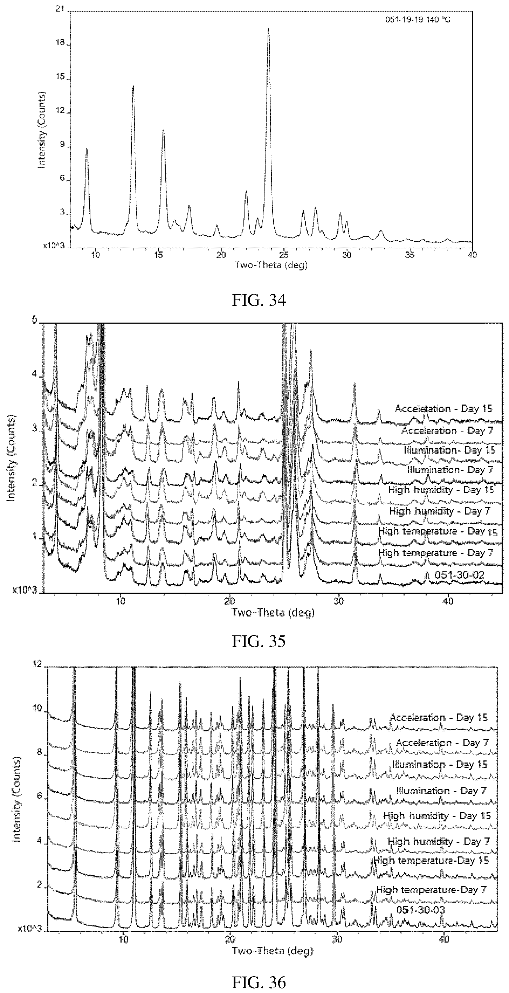

In a comparative embodiment, the present disclosure provides a 7,8-dihydroxyflavone at least having crystal form T. The crystal form T is characterized by an X-ray diffraction pattern thereof, which is characterized by X-ray diffraction peaks at 2θ values of about 9.3 ± 0.2°, 13.0 ± 0.2°, 15.4 ± 0.2°, and 23.8 ± 0.2°. The X-ray diffraction pattern of the crystal form T is shown in FIG. 34. The positions of the characteristic X-ray diffraction peaks of the crystal form T are shown in Table 1 along with the positions of the characteristic X-ray diffraction peaks of the other crystal forms of 7,8-dihydroxyflavone.

The crystal form T can only be obtained by heating the crystal form G to 140°C and cannot exist at room temperature for a long period.

**Table 1**

| Crystal Form | X-ray diffraction peaks (2θ degrees) | | | | | | | | | |
|---|---|---|---|---|---|---|---|---|---|---|
| A | 9.1 | 17.2 | 20.5 | 27.3 | | | | | | |
| B | 6.4 | 9.4 | 10.9 | 12.8 | 13.9 | 17.2 | 18.8 | 20.4 | 20.7 | |
| C | 5.6 | 9.5 | 11.1 | 15.5 | 15.6 | 21.0 | 24.3 | 25.5 | 27.0 | 28.3 |
| E | 14.2 | 16.3 | 20.3 | 23.3 | 24.2 | 27.3 | | | | |
| F | 6.0 | 15.9 | 17.2 | 19.2 | 26.9 | | | | | |
| G | 11.6 | 18.5 | 35.4 | | | | | | | |
| H | 8.3 | 11.2 | 11.6 | 16.7 | 21.5 | | | | | |
| I | 10.5 | 17.0 | 20.5 | | | | | | | |
| J | 4.3 | 5.7 | 6.8 | 8.3 | 12.4 | 12.8 | 17.2 | 18.0 | | |
| K | 10.8 | 19.5 | 22.1 | 22.4 | 24.7 | 28.2 | | | | |
| L | 10.6 | 11.3 | 12.0 | 15.5 | 17.7 | 19.5 | 22.6 | | | |
| M | 8.5 | 14.8 | 16.5 | 19.0 | | | | | | |
| N | 9.3 | 12.3 | 21.1 | 23.8 | 24.6 | 26.6 | | | | |
| O | 3.4 | 5.9 | 8.9 | 10.9 | 11.7 | 13.6 | 14.1 | 15.2 | 20.4 | 20.7 |
| P | 8.3 | 25.0 | 25.9 | 27.4 | 31.4 | | | | | |
| Q | 3.6 | 7.7 | 12.1 | 13.0 | 16.6 | 20.2 | | | | |
| R | 9.7 | 10.8 | 12.4 | 12.8 | 23.2 | | | | | |
| S | 8.6 | 17.3 | 22.6 | | | | | | | |
| T | 9.3 | 13.0 | 15.4 | 23.8 | | | | | | |

In an embodiment, the present disclosure provides a pharmaceutical composition. The pharmaceutical composition comprises a crystal form P of 7,8-dihydroxyflavone and at least one pharmaceutically acceptable excipient. Preferably the pharmaceutical composition is in an oral solid dosage form.

In an embodiment, tablets were prepared according to a standard method and it was found that the crystal form P in tablets could exist stably for 6 months under harsh conditions such as illumination, high temperature and high humidity. The stability of the crystal form P of 7,8-dihydroxyflavone in tablets is not limited by the specific dosage form.

In an embodiment, tablets were prepared according to a standard method and it was found that the crystal form P in tablets had a better dissolution performance.

In an embodiment, the present disclosure further provides a pharmaceutical composition. The pharmaceutical composition comprises a crystal form C of 7,8-dihydroxyflavone and at least one pharmaceutically acceptable excipient. Preferably the pharmaceutical composition is in an oral solid dosage form.

In an embodiment, tablets were prepared according to a standard method and it was found that the crystal form C in tablets could exist stably for 6 months under harsh conditions such as illumination, high temperature and high humidity. The stability of the crystal form C of 7,8-dihydroxyflavone in tablets is not limited by the specific dosage form.

In an embodiment, the present disclosure further provides a pharmaceutical composition. The pharmaceutical composition comprises a crystal form N of 7,8-dihydroxyflavone and at least one pharmaceutically acceptable excipient. Preferably the pharmaceutical composition is in an oral solid dosage form.

In an embodiment, a pharmaceutical composition provided by the present disclosure contains one or more polymorphs of 7,8-dihydroxyflavone described herein. In addition to the active ingredient, the pharmaceutical composition of the present disclosure may further contain one or more excipients. Various excipients may be added to the composition for different purposes.

A filler adds volume to the solid pharmaceutical composition and facilitates the use of pharmaceutical formulations containing the composition for patients and caregivers. The filler for use in the solid composition comprises, for example, microcrystalline cellulose, superfine cellulose, lactose, starch, pregelatinized starch, calcium carbonate, calcium sulfate, sugar, dextrates, dextrin, glucose, calcium hydrogenphosphate dihydrate, calcium phosphate, kaolin, magnesium carbonate, magnesium oxide, maltodextrin, mannitol, polymethacrylate, potassium chloride, powdered cellulose, sodium chloride, sorbitol, and talc.

The solid pharmaceutical composition compressed into dosage forms such as tablets may comprise an excipient; the purpose of using the excipient comprises binding the active ingredient with other excipients together after the compression. The binder for the solid pharmaceutical composition comprises povidone, gum arabic, alginic acid, carbomer, sodium carboxymethyl cellulose, dextrin, ethylcellulose, gelatin, guar gum, hydrogenated vegetable oil, hydroxyethylcellulose, hydroxypropylcellulose, hydroxypropylmethylcellulose, liquid glucose, magnesium aluminium silicate, maltodextrin, methylcellulose, polymethacrylate, polyvinylpyrrolidone, pregelatinized starch, sodium alginate and starch.

The addition of a disintegrant can improve the dissolution rate of the compressed solid pharmaceutical composition in the patient's stomach. The disintegrant comprises sodium carboxymethyl starch, alginic acid, calcium carboxymethyl cellulose, sodium carboxymethyl cellulose, colloidal silicon dioxide, crosslinked sodium carboxymethyl cellulose, crosslinked polyvinylpyrrolidone, guar gum, magnesium aluminium silicate, methylcellulose, microcrystalline cellulose, Polacrilin potassium, powdered cellulose, pregelatinized starch, sodium alginate, sodium starch glycolate, and starch.

When the powdered composition is pressed into a dosage form such as tablets, the composition is subjected to pressure from the die and the punch. Some excipients and the active ingredient are prone to adhesion to the die and the punch on the surface. Such adhesion will result in depressions and other surface irregularities in the product. A lubricant may be added to the composition to reduce adhesion and thereby the product is easy to separate from the die. The lubricant comprises magnesium stearate, calcium stearate, glycerol monostearate, glyceryl palmitostearate, hydrogenated castor oil, hydrogenated vegetable oil, mineral oil, polyethylene glycol, sodium benzoate, sodium dodecyl sulfate, sodium stearyl fumarate, stearic acid, talc, and zinc stearate.

The solid dosage form of the pharmaceutical composition provided by the present disclosure may be, for example, tablets, powders, capsules, suppositories, small capsules, pills, ingots, liquid syrups, suspensions, and the like. In an embodiment, the dosage form of the present disclosure is a tablet.

In one aspect, the present disclosure also provides a method for treating a disease associated with the aberrant tyrosine kinase receptor B (TrkB) signaling pathway such as central nerve injury, peripheral nerve injury, neurodegenerative diseases, psychiatric diseases, hereditary neurological disorders, ophthalmological diseases, metabolic diseases, pain, cardiovascular diseases, tumor, and the like, comprising administering to a subject 7,8-dihydroxyflavone in the crystal forms provided by the present disclosure and at least one pharmaceutically acceptable excipient. In an embodiment, the disease associated with the aberrant TrkB signaling pathway is those resulted from low expression or insufficient activation of TrkB. It is known in the art that 7,8-dihydroxyflavones can be used as a small molecule specific agonist to TrkB to activate the downstream signaling pathway of TrkB.

In one aspect, the present disclosure provides a method for treating or preventing cognitive decline associated with traumatic brain injury or aging, comprising administering to a subject 7,8-dihydroxyflavone in the crystal forms as described.

In one aspect, the present disclosure provides a crystal form P of 7,8-dihydroxyflavone, a crystal form C of 7,8-dihydroxyflavone, or a crystal form N of 7,8-dihydroxyflavone, which can be used in treating or preventing cognitive decline associated with traumatic brain injury or aging.

In one aspect, the present disclosure provides use of the crystal form P of 7,8-dihydroxyflavone, the crystal form C of 7,8-dihydroxyflavone, or the crystal form N of 7,8-dihydroxyflavone in treating or preventing cognitive decline associated with traumatic brain injury or aging. It is known to those skilled in the art that the peak intensity and/or peak condition of X-ray powder diffraction may varies according to the experimental conditions. Also, the measured 2θ values may have an error of about ± 0.2 degrees due to the different accuracy of the instruments. The relative intensity values of the peaks are more obvious than the peak positions to depend on certain properties of the sample, such as crystal size and purity, and therefore the measured peak intensities may show a deviation of about ±20%. Despite the presence of experimental errors, instrumental errors, orientation preferences, etc., a person skilled in the art can obtain sufficient information to identify each crystal form based on the X-ray powder diffraction data provided by the present disclosure.

In the present disclosure, the expression "at least having '#' crystal form" (wherein '#' is a letter) refers to that the 7,8-dihydroxyflavone at least has X-ray characteristic peaks of the crystal form '#'.

In the present disclosure, the "solvent volatilization" refers to the crystal precipitation method commonly used in the field of crystallization, wherein the solution is volatilized continuously to bring the solution from an unsaturated state to a supersaturated state, resulting in the precipitation of crystals.

Each of the above preferred conditions, on the basis of not violating the common knowledge in the field, can be arbitrarily combined.

Unless otherwise specified, the reagents and materials used in the present disclosure are commercially available.

### Examples

The present disclosure is further described below in terms of embodiments, but the present disclosure is not limited to the scope of the embodiments. The experiments for which specific conditions are not specified in the following embodiments are selected in accordance with conventional methods and conditions, or in accordance with the product specifications.

### Test Methods

### Nuclear magnetic analysis (¹H NMR)

Several milligrams of the solid sample were dissolved in a dimethyl sulfoxide-d₆ solvent and subjected to NMR analysis on a Bruker AVANCE NEO 400 (Bruker, GER).

### X-ray powder diffraction (XRPD)

The solid sample obtained from the experiment was analyzed with an X-ray powder diffractometer, Bruker D8 Advance (Bruker, GER). The 2θ scanning angle was from 3° to 45°, the scanning step was 0.02° and the exposure time was 0.08 seconds. The tube voltage and current for testing the sample were 40 kV and 40 mA, respectively, and the sample disk was a zero-background sample disk.

### Thermogravimetric analysis (TGA)

Thermogravimetric analyzer is TA Discovery 55 (TA, US). The sample of 2-5 mg was placed in a balanced open aluminum sample pan and weighed automatically in a TGA heated oven. The sample was heated to a final temperature at a rate of 10°C/min with a nitrogen purge rate of 60 mL/min at the sample and 40 mL/min at the balance.

### Differential Scanning Calorimetry (DSC)

Differential scanning calorimetry analyzer is TA Discovery 2500 (TA, US). The sample of 1-2 mg was accurately weighed and placed in a DSC Tzero sample pan with holes pierced, and heated to a final temperature at a rate of 10°C/min with a nitrogen purge rate of 50 mL/min in the oven.

### Dynamic vapor sorption analysis (DVS)

Dynamic vapor sorption and desorption analysis was measured using DVS Intrinsic (SMS, UK). The test was performed in gradient mode with a humidity change of 50%-95%-0%-50%, with a change of 10% per humidity gradient in the range of 0% to 90%. The gradient endpoint was determined by dm/dt; the gradient endpoint lies where the dm/dt was less than 0.002% and maintained for 10 minutes. After the test was completed, the samples were analyzed by XRPD to confirm whether the solid morphology had changed. The evaluation criteria are shown in Table 2:

**Table 2**

| Classification of water sorption behavior | Weight gain by water sorption |
|---|---|
| Deliquesce | Turn to liquid by absorbing sufficient water |
| Extremely hygroscopic | Weight gain of not less than 15% by water sorption |
| Hygroscopic | Weight gain of less than 15% but not less than 2% by water sorption |
| Slightly hygroscopic | Weight gain of less than 2% but not less than 0.2% by water sorption |
| No goods almost no water sorption | Weight gain of less than 0.2% by water sorption |

### Polarized light microscopy analysis (PLM)

The polarizing microscope is Nikon Ci-POL (Nikon, JPN). A small amount of sample was placed on a slide and a suitable lens was selected to observe the sample morphology.

### Example 1 Preparation of crystal form P of 7,8-dihydroxyflavone

The 20.0 mg of 7,8-dihydroxyflavone was added to 1.0 mL of ethanol and 1.0 mL of ethyl acetate until a suspension was formed, and after the suspension was stirred at 50°C for 7 days, the suspension was centrifuged and a solid obtained was dried in vacuum at room temperature.

Upon the XRPD analysis, the X-ray powder diffraction had diffraction peaks at 4.1 ± 0.2°, 6.2 ± 0.2°, 7.0 ± 0.2°, 7.3 ± 0.2°, 7.9 ± 0.2°, 8.3 ± 0.2°, 11.0 ± 0.2°, 12.5 ± 0.2°, 13.6 ± 0.2°, 14.0 ± 0.2°, 15.8 ± 0.2°, 16.6 ± 0.2°. 17.2 ± 0.2°, 18.4 ± 0.2°, 18.7 ± 0.2°, 19.4 ± 0.2°, 20.8 ± 0.2°, 21.4 ± 0.2°, 22.8 ± 0.2°, 24.1 ± 0.2°, 25.0 ± 0.2°, 25.9 ± 0.2°, 27.0 ± 0.2°, 27.4 ± 0.2°, 31.2 ± 0.2°, 31.4 ± 0.2°, and 33.7 ± 0.2° expressed in 2θ angles. The XRPD pattern is shown in FIG. 1.

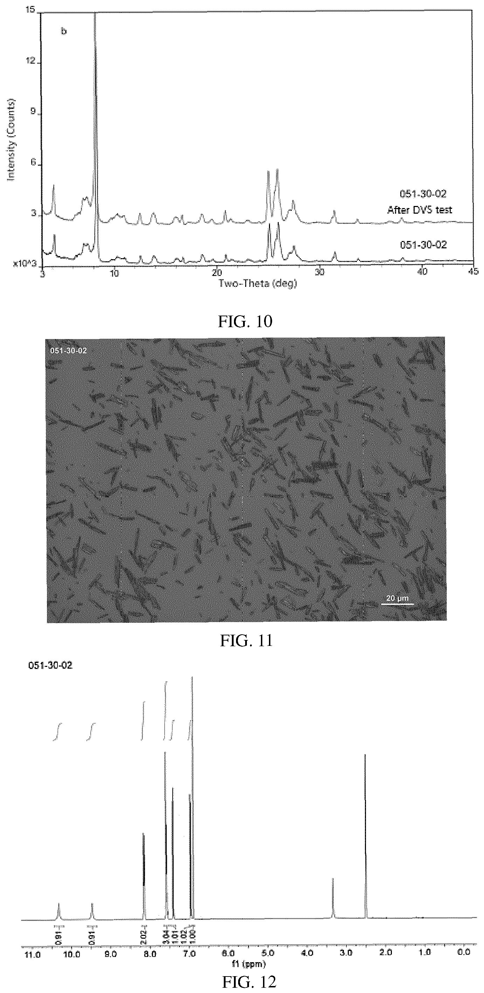

Upon the ¹H NMR analysis, no obvious solvent residue peaks were observed, indicating that the crystal form P of 7,8-dihydroxyflavone was not a solvate. The ¹H NMR spectrum is shown in FIG. 12.

Upon the TGA analysis, the crystal form P of 7,8-dihydroxyflavone did not have obvious weight loss. The TGA curve is shown in FIG. 4.

Upon the DSC analysis, the crystal form P of 7,8-dihydroxyflavone did not have endothermic signal corresponding to weight loss. The DSC curve is shown in FIG. 4.

Upon the DVS analysis, in the dynamic vapor sorption plot of crystal form P of 7,8-dihydroxyflavone, the weight gain was less than 0.1% in the range of 0%-95% relative humidity. The DVS plot is shown in FIG. 5.

Upon the PLM analysis, the crystal form P of 7,8-dihydroxyflavone was fine particles with a particle size generally less than 20 µm. The PLM image is shown in FIG. 11.

### Example 2 Preparation of crystal form C of 7,8-dihydroxyflavone

About 20 mg of the raw material was weighed out, added to an EP tube, added with a certain amount of solvent in batches at room temperature (~25°C), stirred and subjected to ultrasonic treatment until the solid was completely dissolved. The resulting clarified solution was left to stand without cover at room temperature until the solvent volatilized completely or mostly to obtain the solid.

Upon the XRPD analysis, the X-ray powder diffraction had diffraction peaks at 5.6 ± 0.2°, 9.5 ± 0.2°, 11.1 ± 0.2°, 12.7 ± 0.2°, 13.5 ± 0.2°, 13.7 ± 0.2°, 15.5 ± 0.2°, 16.0 ± 0.2°, 16.3 ± 0.2°, 16.6 ± 0.2°, 16.9 ± 0.2°, 17.4± 0.2°, 18.3 ± 0.2°, 18.9 ± 0.2°, 19.2 ± 0.2°, 19.4 ± 0.2°, 20.4 ± 0.2°, 20.8 ± 0.2°, 21.0 ± 0.2°, 21.9 ± 0.2°, 22.2 ± 0.2°, 23.2 ± 0.2°, 23.4 ± 0.2°, 24.1 ± 0.2°, 24.3 ± 0.2°, 25.0 ± 0.2°. 25.2 ± 0.2°, 25.5 ± 0.2°, 25.8 ± 0.2°, 27.0 ± 0.2°, 27.3 ± 0.2°, 27.6 ± 0.2°, 27.9 ± 0.2°, 28.3 ± 0.2°, 28.6 ± 0.2°, 28.9 ± 0.2°, 29.7 ± 0.2°, 30.4 ± 0.2°, 30.7 ± 0.2°, 31.7 ± 0.2°, 33.2 ± 0.2°, 33.6 ± 0.2°, 34.3 ± 0.2°, 34.5 ± 0.2°, 34.6 ± 0.2°, 35.1 ± 0.2°, 35.7 ± 0.2°, 36.3 ± 0.2°, 37.7 ± 0.2°, 39.8 ± 0.2°, 42.6 ± 0.2°, and 44.5 ± 0.2° expressed in 2θ angles. The XRPD pattern is shown in FIG. 2.

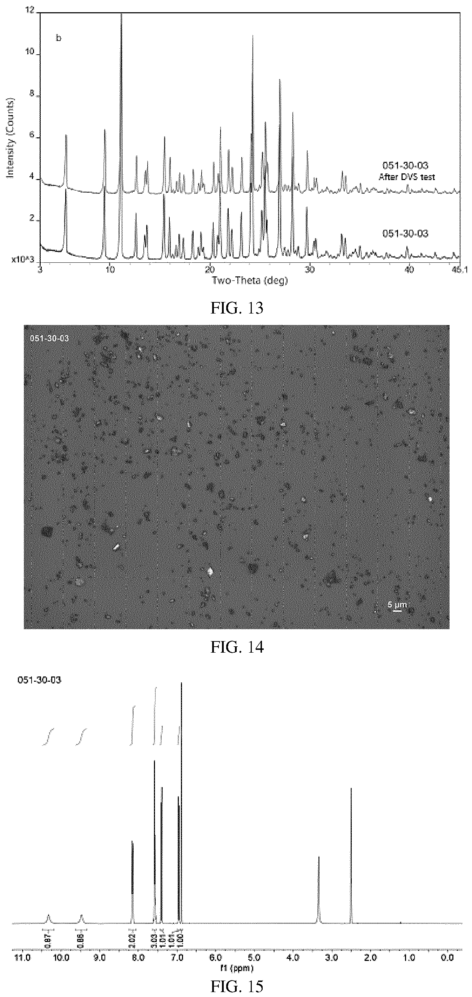

Upon the ¹H NMR analysis, no obvious solvent residue peaks were observed, indicating that the crystal form C of 7,8-dihydroxyflavone was not a solvate. The ¹H NMR spectrum is shown in FIG. 15.

Upon the TGA analysis, the crystal form C of 7,8-dihydroxyflavone had 3.7% weight loss at 150°C relative to the weight before weight loss; such weight loss was the weight of water in the hydrate crystal. The TGA curve is shown in FIG. 6.

Upon the DSC analysis, the crystal form C of 7,8-dihydroxyflavone had endothermic signal at 100°C to 160°C corresponding to weight loss and a melting endothermic peak at 247°C. The DSC curve is shown in FIG. 6.

Upon the DVS analysis, in the dynamic vapor sorption plot of crystal form C of 7,8-dihydroxyflavone, the weight gain was less than 0.1% in the range of 0%-95% relative humidity. The DVS plot is shown in FIG. 7.

Upon the PLM analysis, the crystal form C of 7,8-dihydroxyflavone was fine particles with a particle size generally less than 5 µm. The PLM image is shown in FIG. 14.

### Example 3 Preparation of crystal form N of 7,8-dihydroxyflavone

The 20.0 mg of 7,8-dihydroxyflavone was added to 1.0 mL of methanol until a suspension was formed, and after the suspension was stirred at 50°C for 7 days, the suspension was centrifuged and a solid obtained was dried in vacuum at room temperature.

Upon the XRPD analysis, the X-ray powder diffraction had diffraction peaks at 5.7 ± 0.2°, 9.3 ± 0.2°, 11.1 ± 0.2°, 11.4 ± 0.2°, 11.9 ± 0.2°, 12.3 ± 0.2°, 13.1 ± 0.2°, 14.2 ± 0.2°, 14.9 ± 0.2°, 15.9 ± 0.2°, 16.8 ± 0.2°, 17.1± 0.2°, 17.7 ± 0.2°, 18.7 ± 0.2°, 18.9 ± 0.2°, 19.2 ± 0.2°, 20.2 ± 0.2°, 20.7 ± 0.2°, 21.1 ± 0.2°, 22.1 ± 0.2°, 22.4 ± 0.2°, 23.1 ± 0.2°, 23.2 ± 0.2°, 23.4 ± 0.2°, 23.8 ± 0.2°, 24.6 ± 0.2°. 25.2 ± 0.2°, 25.7 ± 0.2°, 26.3 ± 0.2°, 26.6 ± 0.2°, 26.9 ± 0.2°, 27.3 ± 0.2°, 28.9 ± 0.2°, 28.0 ± 0.2°, 29.0 ± 0.2°, 29.4 ± 0.2°, 29.6 ± 0.2°, 30.4 ± 0.2°, 31.3 ± 0.2°, 31.9 ± 0.2°, 32.1 ± 0.2°, 32.4 ± 0.2°, 35.0 ± 0.2°, 35.4 ± 0.2°, 36.2 ± 0.2°, 36.8 ± 0.2°, 38.2 ± 0.2°, 38.4 ± 0.2°, 38.6 ± 0.2°, 38.9 ± 0.2°, 41.0 ± 0.2°, 41.4 ± 0.2°, 42.0 ± 0.2°, and 43.9 ± 0.2° expressed in 2θ angles. The XRPD pattern is shown in FIG. 3.

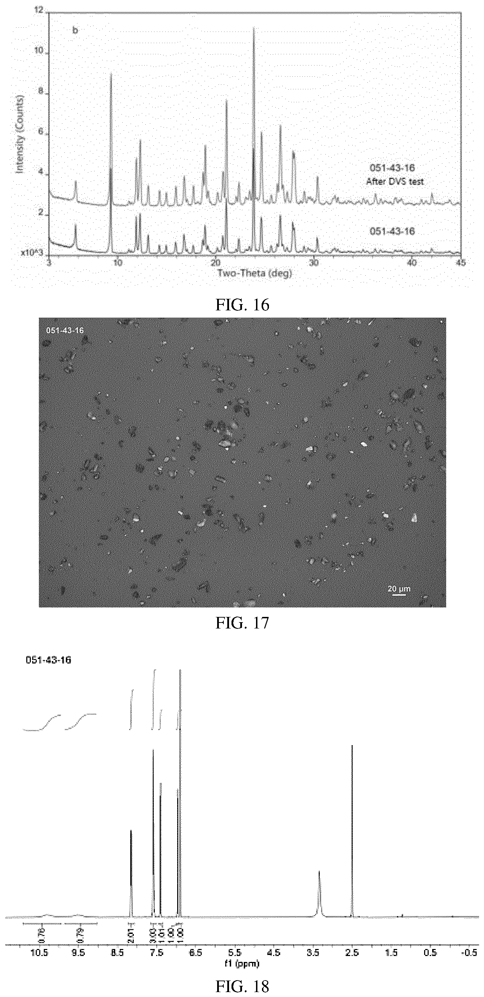

Upon the ¹H NMR analysis, no obvious solvent residue peaks were observed, indicating that the crystal form N of 7,8-dihydroxyflavone was not a solvate. The ¹H NMR spectrum is shown in FIG. 18.

Upon the TGA analysis, the crystal form N of 7,8-dihydroxyflavone had 6.3% weight loss at 40°C to 80°C relative to the weight before weight loss; such weight loss was the weight of water in the hydrate crystal. The TGA curve is shown in FIG. 8.

Upon the DSC analysis, the crystal form N of 7,8-dihydroxyflavone had an endothermic peak at 100°C corresponding to weight loss and exothermic signals at 136°C and 169°C, respectively. The DSC curve is shown in FIG. 8.

Upon the DVS analysis, in the dynamic vapor sorption plot of crystal form N of 7,8-dihydroxyflavone, the weight gain was less than 0.1% in the range of 0%-95% relative humidity. The DVS plot is shown in FIG. 9.

Upon the PLM analysis, the crystal form N of 7,8-dihydroxyflavone was fine particles with a particle size generally less than 20 µm. The PLM image is shown in FIG. 17.

### Comparative Example 1 Preparation of crystal form A of 7,8-dihydroxyflavone

The 20.0 mg of 7,8-dihydroxyflavone was added to 2.0 mL of ethanol until a suspension was formed, and after the suspension was stirred at room temperature for 7 days, the suspension was centrifuged and a solid obtained was dried in vacuum at room temperature.

Upon the XRPD analysis, the X-ray powder diffraction had diffraction peaks at 5.6 ± 0.2°, 9.1 ± 0.2°, 11.7 ± 0.2°, 12.0 ± 0.2°, 12.2 ± 0.2°, 16.4 ± 0.2°, 17.2 ± 0.2°, 18.2 ± 0.2°, 18.5 ± 0.2°, 20.4 ± 0.2°, 20.5 ± 0.2°, 21.0 ± 0.2°, 23.5 ± 0.2°, 26.1 ± 0.2°, 27.3 ± 0.2°, 27.8 ± 0.2°, 35.3 ± 0.2°, and 41.1 ± 0.2° expressed in 2θ angles. The XRPD pattern is shown in FIG. 19.

### Comparative Example 2 Preparation of crystal form B of 7,8-dihydroxyflavone

The 40.0 mg of 7,8-dihydroxyflavone was added to 9.0 mL of ethyl formate until a suspension was formed, and after the suspension was stirred at room temperature for 7 days, the suspension was centrifuged and a solid obtained was dried in vacuum at room temperature.

Upon the XRPD analysis, the X-ray powder diffraction had diffraction peaks at 3.1 ± 0.2°, 3.4 ± 0.2°, 3.7 ± 0.2°, 5.5 ± 0.2°, 5.9 ± 0.2°, 6.4 ± 0.2°, 7.3 ± 0.2°, 8.2 ± 0.2°, 8.5 ± 0.2°, 9.4 ± 0.2°, 9.8 ± 0.2°, 10.3 ± 0.2°, 10.9± 0.2°, 11.2 ± 0.2°, 11.8 ± 0.2°, 12.8 ± 0.2°, 13.4 ± 0.2°, 13.9 ± 0.2°, 14.2 ± 0.2°, 14.8 ± 0.2°, 15.2 ± 0.2°, 15.7 ± 0.2°, 16.3 ± 0.2°, 16.7 ± 0.2°, 17.2 ± 0.2°, 17.9 ± 0.2°, 18.8 ± 0.2°. 19.3 ± 0.2°, 19.8 ± 0.2°, 20.4 ± 0.2°, 20.7 ± 0.2°, 22.1 ± 0.2°, 22.2 ± 0.2°, 22.5 ± 0.2°, 23.7 ± 0.2°, 24.2 ± 0.2°, 24.9 ± 0.2°, 25.3 ± 0.2°, 26.0 ± 0.2°, 26.7 ± 0.2°, 27.8 ± 0.2°, 28.1 ± 0.2°, 28.3 ± 0.2°, 29.0 ± 0.2°, 30.2 ± 0.2°, 32.5 ± 0.2°, 34.7 ± 0.2°, and 41.46 ± 0.2° expressed in 2θ angles. The XRPD pattern is shown in FIG. 20.

### Comparative Example 3 Preparation of crystal form E of 7,8-dihydroxyflavone

About 20 mg of the raw material was weighed out, added to an EP tube, added with 2 mL of ethylene glycol dimethyl ether at room temperature (~25°C), stirred and subjected to ultrasonic treatment until the solid was completely dissolved. The resulting clarified solution was left to stand without cover at room temperature until the solvent volatilized completely or mostly to obtain the solid.

Upon the XRPD analysis, the X-ray powder diffraction had diffraction peaks at 5.6 ± 0.2°, 8.9 ± 0.2°, 11.1 ± 0.2°, 11.6 ± 0.2°, 11.9 ± 0.2°, 12.1 ± 0.2°, 13.5 ± 0.2°, 14.0 ± 0.2°, 14.2 ± 0.2°, 15.8 ± 0.2°, 16.3 ± 0.2°, 16.6± 0.2°, 17.0 ± 0.2°, 17.3 ± 0.2°, 17.9 ± 0.2°, 18.3 ± 0.2°, 18.6 ± 0.2°, 18.8 ± 0.2°, 19.0 ± 0.2°, 19.3 ± 0.2°, 20.3 ± 0.2°, 21.2 ± 0.2°, 21.7 ± 0.2°, 22.0 ± 0.2°, 22.3 ± 0.2°, 22.6 ± 0.2°. 23.3 ± 0.2°, 23.8 ± 0.2°, 24.2 ± 0.2°, 25.0 ± 0.2°, 25.4 ± 0.2°, 25.8 ± 0.2°, 26.5 ± 0.2°, 26.9 ± 0.2°, 27.3 ± 0.2°, 27.8 ± 0.2°, 28.1 ± 0.2°, 28.6 ± 0.2°, 29.2 ± 0.2°, 29.7 ± 0.2°, 30.7 ± 0.2°, 31.8 ± 0.2°, 31.9 ± 0.2°, 34.0 ± 0.2°, 34.8 ± 0.2°, 34.9 ± 0.2°, 35.5 ± 0.2°, 36.2 ± 0.2°, 37.6 ± 0.2°, 39.5 ± 0.2°, 40.6 ± 0.2°, 42.6 ± 0.2°, and 43.2 ± 0.2° expressed in 2θ angles. The XRPD pattern is shown in FIG. 21.

### Comparative Example 4 Preparation of crystal form F of 7,8-dihydroxyflavone

The 20.0 mg of 7,8-dihydroxyflavone was added to 10.0 mL of acetonitrile until a suspension was formed, and after the suspension was stirred at room temperature for 7 days, the suspension was centrifuged and a solid obtained was dried in vacuum at room temperature.

Upon the XRPD analysis, the X-ray powder diffraction had diffraction peaks at 6.0 ± 0.2°, 10.3 ± 0.2°, 12.0 ± 0.2°, 12.3 ± 0.2°, 13.7 ± 0.2°, 15.0 ± 0.2°, 15.9 ± 0.2°, 17.2 ± 0.2°, 18.2 ± 0.2°, 19.2 ± 0.2°, 20.9 ± 0.2°, 21.9± 0.2°, 22.7 ± 0.2°, 24.2 ± 0.2°, 24.7 ± 0.2°, 25.5 ± 0.2°, 26.2 ± 0.2°, 26.9 ± 0.2°, 27.6 ± 0.2°, 28.2 ± 0.2°, 29.5 ± 0.2°, 30.1 ± 0.2°, 31.0 ± 0.2°, 32.0 ± 0.2°, 33.3 ± 0.2°, 33.7 ± 0.2°, 35.5 ± 0.2°, 36.4 ± 0.2°, 36.9 ± 0.2°, 38.0 ± 0.2°, and 38.5 ± 0.2° expressed in 2θ angles. The XRPD pattern is shown in FIG. 22.

### Comparative Example 5 Preparation of crystal form G of 7,8-dihydroxyflavone

The 160 mg of 7,8-dihydroxyflavone was weighed, and 11 mL of ethylene glycol dimethyl ether was added dropwise at room temperature to dissolve the sample completely; 1.3 mL of the solution was taken, and added dropwise to 6.5 mL of water. After the system was stirred for 15 min to 1 h, a precipitated solid was separated by centrifugation, and the solid was dried in vacuum at room temperature.

Upon the XRPD analysis, the X-ray powder diffraction had diffraction peaks at 11.6 ± 0.2°, 12.1 ± 0.2°, 13.1 ± 0.2°, 13.5 ± 0.2°, 16.8 ± 0.2°, 18.5 ± 0.2°, 20.3 ± 0.2°, 21.3 ± 0.2°, 21.7 ± 0.2°, 22.9 ± 0.2°, 23.4 ± 0.2°, 24.2 ± 0.2°, 24.7 ± 0.2°, 26.5 ± 0.2°, 26.8 ± 0.2°, 28.2 ± 0.2°, 30.0 ± 0.2°, 31.9 ± 0.2°, 34.2 ± 0.2°, 35.4 ± 0.2°, 38.9 ± 0.2°, and 42.0 ± 0.2° expressed in 2θ angles. The XRPD pattern is shown in FIG. 23.

### Comparative Example 6 Preparation of crystal form H of 7,8-dihydroxyflavone

The 160 mg of 7,8-dihydroxyflavone was weighed, and 11 mL of ethylene glycol dimethyl ether was added dropwise at room temperature to dissolve the sample completely; 1.3 mL of the solution was taken, and added dropwise to 1.0 mL of n-heptane. After the system was stirred for 15 min to 1 h at room temperature, a precipitated solid was separated by centrifugation, and the solid was dried in vacuum at room temperature.

Upon the XRPD analysis, the X-ray powder diffraction had diffraction peaks at 7.2 ± 0.2°, 8.3 ± 0.2°, 8.8 ± 0.2°, 9.5 ± 0.2°, 10.2 ± 0.2°, 11.2 ± 0.2°, 11.6 ± 0.2°, 12.2 ± 0.2°, 12.4 ± 0.2°, 13.4 ± 0.2°, 13.7 ± 0.2°, 14.5 ± 0.2°, 14.9 ± 0.2°, 15.5 ± 0.2°, 16.2 ± 0.2°, 16.7 ± 0.2°, 18.4 ± 0.2°, 19.1 ± 0.2°, 19.2 ± 0.2°, 19.9 ± 0.2°, 20.3 ± 0.2°, 20.9 ± 0.2°, 21.5 ± 0.2°, 21.9 ± 0.2°, 23.1 ± 0.2°, 23.9 ± 0.2°. 25.5 ± 0.2°, 25.8 ± 0.2°, 27.6 ± 0.2°, 27.7 ± 0.2°, 28.0 ± 0.2°, 28.9 ± 0.2°, and 31.0 ± 0.2° expressed in 2θ angles. The XRPD pattern is shown in FIG. 24.

### Comparative Example 7 Preparation of crystal form I of 7,8-dihydroxyflavone

The 160 mg of 7,8-dihydroxyflavone was weighed, and 11 mL of ethylene glycol dimethyl ether was added dropwise at room temperature to dissolve the sample completely; 1.3 mL of the solution was taken, and added dropwise to 5.0 mL of water. After the system was stirred for 15 min to 1 h at room temperature, a precipitated solid was separated by centrifugation, and the solid was dried in vacuum at room temperature.

Upon the XRPD analysis, the X-ray powder diffraction had diffraction peaks at 7.0 ± 0.2°, 8.3 ± 0.2°, 10.5 ± 0.2°, 12.3 ± 0.2°, 13.0 ± 0.2°, 14.0 ± 0.2°, 14.8 ± 0.2°, 16.7 ± 0.2°, 17.0 ± 0.2°, 20.5 ± 0.2°, 21.8 ± 0.2°, 22.0 ± 0.2°, 22.1 ± 0.2°, 23.8 ± 0.2°, 25.9 ± 0.2°, 27.1 ± 0.2°, 27.6 ± 0.2°, and 33.7 ± 0.2° expressed in 2θ angles. The XRPD pattern is shown in FIG. 25.

### Comparative Example 8 Preparation of crystal form J of 7,8-dihydroxyflavone

The 20.0 mg of 7,8-dihydroxyflavone was added to 10.0 mL of toluene until a suspension was formed, and after the suspension was stirred at room temperature for 7 days, the suspension was centrifuged and a solid obtained was dried in vacuum at room temperature.

Upon the XRPD analysis, the X-ray powder diffraction had diffraction peaks at 4.3 ± 0.2°, 5.7 ± 0.2°, 6.8 ± 0.2°, 8.3 ± 0.2°, 10.6 ± 0.2°, 10.8 ± 0.2°, 11.4 ± 0.2°, 12.4 ± 0.2°, 12.8 ± 0.2°, 13.9 ± 0.2°, 15.1 ± 0.2°, 16.0 ± 0.2°, 16.7 ± 0.2°, 17.1 ± 0.2°, 18.0 ± 0.2°, 19.2 ± 0.2°, 20.1 ± 0.2°, 20.5 ± 0.2°, 21.2 ± 0.2°, 21.7 ± 0.2°, 22.9 ± 0.2°, 24.0 ± 0.2°, 24.2 ± 0.2°, 25.1 ± 0.2°, 26.3 ± 0.2°, 26.9 ± 0.2°, 28.0 ± 0.2°, 28.6 ± 0.2°, and 32.1 ± 0.2° expressed in 2θ angles. The XRPD pattern is shown in FIG. 26.

### Comparative Example 9 Preparation of crystal form K of 7,8-dihydroxyflavone

The 20.0 mg of 7,8-dihydroxyflavone was added to 2.0 mL of dioxane until a suspension was formed, and after the suspension was stirred at room temperature for 7 days, the suspension was centrifuged and a solid obtained was dried in vacuum at room temperature.

Upon the XRPD analysis, the X-ray powder diffraction had diffraction peaks at 9.9 ± 0.2°, 10.8 ± 0.2°, 12.0 ± 0.2°, 12.4 ± 0.2°, 13.4 ± 0.2°, 14.3 ± 0.2°, 14.7 ± 0.2°, 15.7 ± 0.2°, 17.5 ± 0.2°, 17.9 ± 0.2°, 19.5 ± 0.2°, 20.4± 0.2°, 20.7 ± 0.2°, 20.9 ± 0.2°, 22.1 ± 0.2°, 22.4 ± 0.2°, 23.4 ± 0.2°, 23.9 ± 0.2°, 24.7 ± 0.2°, 25.8 ± 0.2°, 26.3 ± 0.2°, 27.3 ± 0.2°, 27.4 ± 0.2°, 28.1 ± 0.2°, 28.7 ± 0.2°, 29.6 ± 0.2°. 30.4 ± 0.2°, 31.0 ± 0.2°, 31.4 ± 0.2°, 31.9 ± 0.2°, 32.4 ± 0.2°, 33.1 ± 0.2°, 33.7 ± 0.2°, 34.2 ± 0.2°, 34.8 ± 0.2°, 35.4 ± 0.2°, 36.2 ± 0.2°, 37.0 ± 0.2°, 37.8 ± 0.2°, 38.8 ± 0.2°, 39.6 ± 0.2°, 40.9 ± 0.2°, 43.8 ± 0.2°, and 44.8 ± 0.2° expressed in 2θ angles. The XRPD pattern is shown in FIG. 27.

### Comparative Example 10 Preparation of crystal form L of 7,8-dihydroxyflavone

The 20.0 mg of 7,8-dihydroxyflavone was added to 10.0 mL of methyl formate until a suspension was formed, and after the suspension was stirred at room temperature for 7 days, the suspension was centrifuged and a solid obtained was dried in vacuum at room temperature.

Upon the XRPD analysis, the X-ray powder diffraction had diffraction peaks at 7.7 ± 0.2°, 10.6 ± 0.2°, 11.3 ± 0.2°, 12.0 ± 0.2°, 12.4 ± 0.2°, 15.5 ± 0.2°, 16.9 ± 0.2°, 17.7 ± 0.2°, 18.9 ± 0.2°, 19.5 ± 0.2°, 20.0 ± 0.2°, 21.0 ± 0.2°, 21.3 ± 0.2°, 22.0 ± 0.2°, 22.2 ± 0.2°, 22.6 ± 0.2°, 23.3 ± 0.2°, 23.7 ± 0.2°, 25.5 ± 0.2°, 25.6 ± 0.2°, 26.3 ± 0.2°, 27.3 ± 0.2°, 27.8 ± 0.2°, 28.9 ± 0.2°, 29.3 ± 0.2°, 30.4 ± 0.2°. 31.6 ± 0.2°, 32.1 ± 0.2°, 32.3 ± 0.2°, 32.4 ± 0.2°, 34.2 ± 0.2°, 34.9 ± 0.2°, and 37.9 ± 0.2° expressed in 2θ angles. The XRPD pattern is shown in FIG. 28.

### Comparative Example 11 Preparation of crystal form M of 7,8-dihydroxyflavone

The 20.0 mg of 7,8-dihydroxyflavone was added to 2.0 mL of acetone until a suspension was formed, and after the suspension was stirred at room temperature for 7 days, the suspension was centrifuged and a solid obtained was dried in vacuum at room temperature.

Upon the XRPD analysis, the X-ray powder diffraction had diffraction peaks at 8.5 ± 0.2°, 11.1 ± 0.2°, 14.5 ± 0.2°, 14.8 ± 0.2°, 16.5 ± 0.2°, 18.1 ± 0.2°, 18.3 ± 0.2°, 19.0 ± 0.2°, 20.8 ± 0.2°, 22.0 ± 0.2°, 22.3 ± 0.2°, 22.8 ± 0.2°, 14.2 ± 0.2°, 24.4 ± 0.2°, 25.4 ± 0.2°, 25.9 ± 0.2°, 26.4 ± 0.2°, 26.6 ± 0.2°, 27.7 ± 0.2°, 28.3 ± 0.2°, 29.0 ± 0.2°, 30.5 ± 0.2°, 30.9 ± 0.2°, 32.5 ± 0.2°, and 33.3 ± 0.2° expressed in 2θ angles. The XRPD pattern is shown in FIG. 29.

### Comparative Example 12 Preparation of crystal form O of 7,8-dihydroxyflavone

The 20.0 mg of 7,8-dihydroxyflavone was added to 1.0 mL of chloroform until a suspension was formed, and after the suspension was stirred at 50°C for 7 days, the suspension was centrifuged and a solid obtained was dried in vacuum at room temperature.

Upon the XRPD analysis, the X-ray powder diffraction had diffraction peaks at 3.4 ± 0.2°, 5.9 ± 0.2°, 7.0 ± 0.2°, 8.5 ± 0.2°, 8.9 ± 0.2°, 10.3 ± 0.2°, 10.9 ± 0.2°, 11.7 ± 0.2°, 12.8 ± 0.2°, 13.6 ± 0.2°, 14.1 ± 0.2°, 14.7 ± 0.2°. 15.2 ± 0.2°, 16.0 ± 0.2°, 17.0 ± 0.2°, 17.3 ± 0.2°, 17.9 ± 0.2°, 18.6 ± 0.2°, 19.3 ± 0.2°, 19.8 ± 0.2°, 20.4 ± 0.2°, 20.7 ± 0.2°, 22.1 ± 0.2°, 22.6 ± 0.2°, 23.9 ± 0.2°, 25.1 ± 0.2°, 25.5 ± 0.2°, 25.7 ± 0.2°, 27.1 ± 0.2°, 27.3 ± 0.2°, 27.5 ± 0.2°, 28.3 ± 0.2°, 28.9 ± 0.2°, 29.5 ± 0.2°, 29.7 ± 0.2°, 29.8 ± 0.2°, 32.6 ± 0.2°, 32.7 ± 0.2°, 36.2 ± 0.2°, 41.3 ± 0.2°, and 41.4 ± 0.2° expressed in 2θ angles. The XRPD pattern is shown in FIG. 30.

### Comparative Example 13 Preparation of crystal form Q of 7,8-dihydroxyflavone

The 20.0 mg of 7,8-dihydroxyflavone was added to 0.2 mL of dioxane and 1.0 mL of chloroform until a suspension was formed, and after the suspension was stirred at 50°C for 7 days, the suspension was centrifuged and a solid obtained was dried in vacuum at room temperature.

Upon the XRPD analysis, the X-ray powder diffraction had diffraction peaks at 3.2 ± 0.2°, 3.6 ± 0.2°, 4.5 ± 0.2°, 5.6 ± 0.2°, 6.1 ± 0.2°, 7.3 ± 0.2°, 7.7 ± 0.2°, 8.8 ± 0.2°, 9.4 ± 0.2°, 9.7 ± 0.2°, 10.9 ± 0.2°, 11.2 ± 0.2°, 12.1± 0.2°, 13.0 ± 0.2°, 13.9 ± 0.2°, 14.5 ± 0.2°, 15.3 ± 0.2°, 15.5 ± 0.2°, 16.2 ± 0.2°, 16.6 ± 0.2°, 17.3 ± 0.2°, 17.7 ± 0.2°, 18.1 ± 0.2°, 18.5 ± 0.2°, 19.2 ± 0.2°, 19.6 ± 0.2°, 19.9 ± 0.2°. 20.2 ± 0.2°, 20.6 ± 0.2°, 21.2 ± 0.2°, 21.9 ± 0.2°, 22.7 ± 0.2°, 23.9 ± 0.2°, 24.2 ± 0.2°, 25.9 ± 0.2°, 26.9 ± 0.2°, 27.6 ± 0.2°, 29.6 ± 0.2°, 33.2 ± 0.2°, 35.2 ± 0.2°, and 40.8 ± 0.2° expressed in 2θ angles. The XRPD pattern is shown in FIG. 31.

### Comparative Example 14 Preparation of crystal form R of 7,8-dihydroxyflavone

The crystal form N was heated to 100°C by in-situ variable temperature XRPD for the preparation.

Upon the XRPD analysis, the X-ray powder diffraction had diffraction peaks at 9.7 ± 0.2°, 10.8 ± 0.2°, 11.1 ± 0.2°, 11.7 ± 0.2°, 12.4 ± 0.2°, 12.6 ± 0.2°, 12.8 ± 0.2°, 13.4 ± 0.2°, 13.9 ± 0.2°, 14.1 ± 0.2°, 14.3 ± 0.2°, 14.5± 0.2°, 15.6 ± 0.2°, 16.1 ± 0.2°, 16.4 ± 0.2°, 17.5 ± 0.2°, 19.2 ± 0.2°, 19.7 ± 0.2°, 21.4 ± 0.2°, 22.2 ± 0.2°, 23.0 ± 0.2°, 23.2 ± 0.2°, 23.4 ± 0.2°, 24.2 ± 0.2°, 24.4 ± 0.2°, 24.7 ± 0.2°. 25.1 ± 0.2°, 25.4 ± 0.2°, 25.9 ± 0.2°, 26.5 ± 0.2°, 27.9 ± 0.2°, 28.5 ± 0.2°, 28.7 ± 0.2°, 29.9 ± 0.2°, 31.5 ± 0.2°, 32.2 ± 0.2°, and 44.3 ± 0.2° expressed in 2θ angles. The XRPD pattern is shown in FIG. 32.

### Comparative Example 15 Preparation of crystal form S of 7,8-dihydroxyflavone

The 160 mg of 7,8-dihydroxyflavone was weighed, and 11 mL of ethylene glycol dimethyl ether was added dropwise at room temperature to dissolve the sample completely; 1.3 mL of the solution was taken, and added dropwise to 8.0 mL of butyl formate. After the system was stirred for 15 min to 1 h at room temperature, a precipitated solid was separated by centrifugation, and the solid was dried in vacuum at room temperature.

Upon the XRPD analysis, the X-ray powder diffraction had diffraction peaks at 8.6 ± 0.2°, 12.6 ± 0.2°, 17.3 ± 0.2°, 18.4 ± 0.2°, 22.6 ± 0.2°, 26.9 ± 0.2°, 29.1 ± 0.2°, 31.3 ± 0.2°, 33.2 ± 0.2°, 35.1 ± 0.2°, 35.7 ± 0.2°, 41.8 ± 0.2°, and 44.8 ± 0.2° expressed in 2θ angles. The XRPD pattern is shown in FIG. 33.

### Comparative Example 16 Preparation of crystal form T of 7,8-dihydroxyflavone

The crystal form G was heated to 140°C by in-situ variable temperature XRPD for the preparation.

Upon the XRPD analysis, the X-ray powder diffraction had diffraction peaks at 8.3 ± 0.2°, 9.3 ± 0.2°, 10.4 ± 0.2°, 10.9 ± 0.2°, 11.4 ± 0.2°, 12.4 ± 0.2°, 13.0 ± 0.2°, 14.0 ± 0.2°, 15.4 ± 0.2°, 16.2 ± 0.2°, 16.7 ± 0.2°, 17.5± 0.2°, 18.5 ± 0.2°, 19.7 ± 0.2°, 20.5 ± 0.2°, 22.0 ± 0.2°, 22.9 ± 0.2°, 23.8 ± 0.2°, 25.2 ± 0.2°, 26.0 ± 0.2°, 26.5 ± 0.2°, 27.5 ± 0.2°, 28.0 ± 0.2°, 29.5 ± 0.2°, 30.0 ± 0.2°, 31.1 ± 0.2°. 31.3 ± 0.2°, 31.5 ± 0.2°, 31.7 ± 0.2°, 32.7 ± 0.2°, 34.0 ± 0.2°, 34.7 ± 0.2°, 34.9 ± 0.2°, 35.8 ± 0.2°, 36.1 ± 0.2°, 38.0 ± 0.2°, 38.2 ± 0.2°, 42.1 ± 0.2°, 43.5 ± 0.2°, 43.7 ± 0.2°, and 44.1 ± 0.2° expressed in 2θ angles. The XRPD pattern is shown in FIG. 34.

### Tablet Preparation Example 1 Preparation of 7,8-dihydroxyflavone tablets

The 80 g of active pharmaceutical ingredient (dihydroxyflavones in the crystal forms prepared by the present disclosure), 80 g of lactose, 13 g of microcrystalline cellulose, 12 g of povidone, and 7 g of sodium carboxymethyl starch were mixed, poured into a 1 L granulation pot, stirred for 6 min, added with 40-50 g of pure water, and subjected to the wet granulation by stirring for 8-10 min. After drying and granulation grading, 7 g of sodium carboxymethyl starch and 1 g of magnesium stearate were added, and the materials were blended totally and then subjected to tableting and demolding to obtain the target tablets.

### Performance Example 1 Stability of different 7,8-dihydroxyflavone crystal forms

The stability of different 7,8-dihydroxyflavonoid crystal forms was studied under high temperature (60°C), high humidity (25°C/92.5% RH), illumination (25°C/4500 Lux), and acceleration (40°C/75% RH) conditions, and samples were taken for XRPD characterization on day 7 and day 15, respectively. The results are shown in Table 3, and the results of the XPRD analysis are shown in FIGS. 35-37.

**Table 3**

| **Crystal form** | **Condition** | **Result on day 7** | **Result on day 15** |
|---|---|---|---|
| Crystal form P | High temperature 60°C | No change | No change |
| | High humidity 25°C/ 92.5% RH | No change | No change |
| | Illumination 25°C/ 4500 Lux | No change | No change |
| | Acceleration 40°C/75% RH | No change | No change |
| Crystal form C | High temperature 60°C | No change | No change |
| | High humidity 25°C/ 92.5% RH | No change | No change |
| | Illumination 25°C/ 4500 Lux | No change | No change |
| | Acceleration 40°C/75% RH | No change | No change |
| Crystal form N | High temperature 60°C | No change | No change |
| | High humidity 25°C/ 92.5% RH | Partially transformed into crystal form C | Partially transformed into crystal form C |
| | Illumination 25°C/ 4500 Lux | Partially transformed into crystal form C | Partially transformed into crystal form C |
| | Acceleration 40°C/75% RH | Partially transformed into crystal form C | Partially transformed into crystal form C |

### Performance Example 2 Dynamic solubility of different 7,8-dihydroxyflavone crystal forms in three biological medium (FaSSIF, FeSSIF and FaSSGF) and water

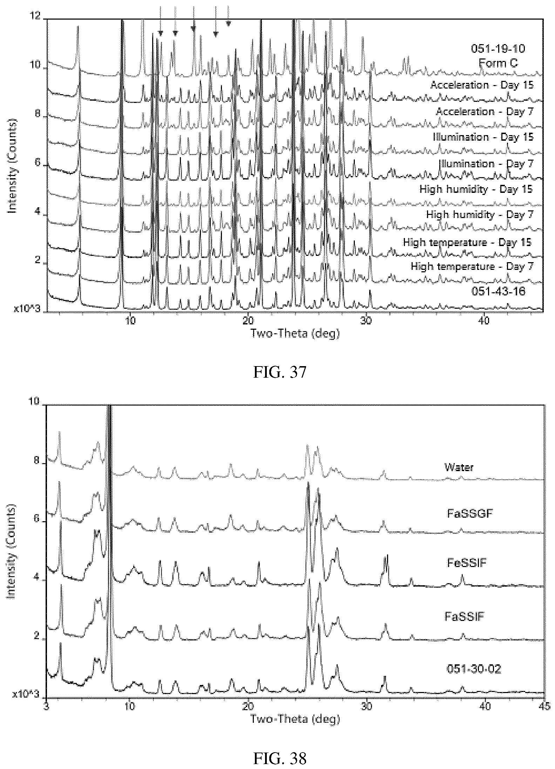

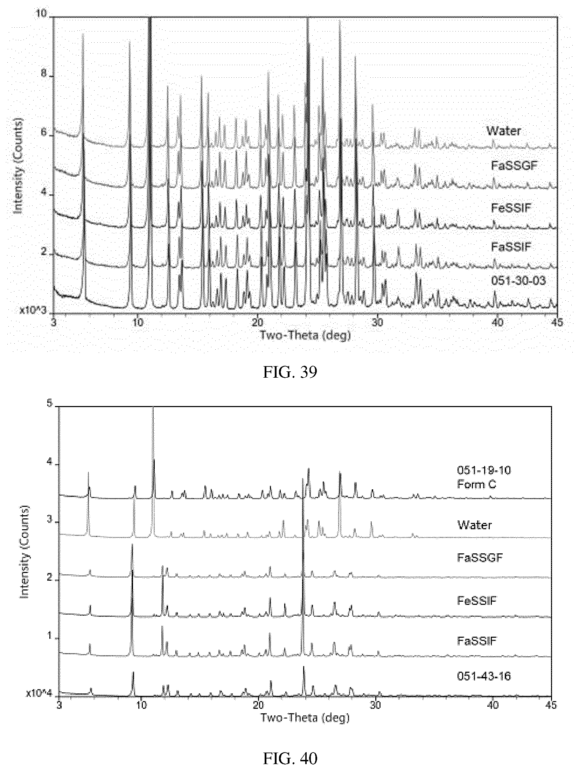

The 20 mg of 7,8-dihydroxyflavones in different crystal forms were weighed out separately and added into 4.0 mL of biological medium or water, and shaken at a constant temperature of 37°C for 24 h. Samples were taken at 0.5 h, 2 h and 24 h, respectively, and the sampled solutions were filtered through 0.22 µm aquo-system filter membranes. Signal peak areas of the solutions were measured by HPLC, and finally, concentrations of the compounds in the solutions were calculated based on the peak areas, HPLC standard curves of the raw materials, and the dilution times. In addition, the supernatant at 24 h was taken to test pH, and the residual solid was subjected to the XRPD test. The preparation of the biological medium is shown in Table 4. The experimental results are shown in Table 5, and the results of the XPRD analysis are shown in FIGS. 38-40.

**Table 4**

| Biological medium | Preparation process |
|---|---|
| FaSSIF | Weigh 2.082 g of FaSSIF concentrated solution and 112.4 mg of FaSSIF/FeSSIF/FaSSGF powder, and add pure water to achieve 50 mL volume in total (pH 6.5). |
| FeSSIF | Weigh 4.070 g of FeSSIF concentrate solution and 560.1 mg of FaSSIF/FeSSIF/FaSSGF powder, and add pure water to achieve 50 mL volume in total (pH 5.0). |
| FaSSGF | Weigh 3.676 g of FaSSGF concentrate solution and 6.00 mg of FaSSIF/FeSSIF/FaSSGF powder, and add pure water to achieve 100 mL volume in total (pH 1.6). |

**Table 5**

| Crystal form | Medium | pH | Sample mass (mg) | Solubility (µg/mL) | | | Final pH | Residual solid XRPD |
|---|---|---|---|---|---|---|---|---|
| | | | | 0.5 h | 2 h | 24 h | | |
| Crystal form P | FaSSIF | 6.5 | 20.2 | 24.3 | 25.9 | 20.5 | 6.4 | Crystal form P |
| | FeSSIF | 5.0 | 20.2 | 74.5 | 74.3 | 71.2 | 5.0 | Crystal form P |
| | FaSSGF | 1.6 | 20.0 | 8.6 | 8.6 | 5.6 | 1.6 | Crystal form P |
| | Water | - | 19.9 | 11.2 | 10.6 | 10.6 | 4.6 | Crystal form P |
| Crystal form C | FaSSIF | 6.5 | 20.5 | 9.0 | 10.2 | 9.8 | 6.5 | Crystal form C |
| | FeSSIF | 5.0 | 20.3 | 33.5 | 33.9 | 33.2 | 5.0 | Crystal form C |
| | FaSSGF | 1.6 | 20.4 | 2.5 | 2.2 | 2.4 | 1.6 | Crystal form C |
| | Water | - | 20.2 | 2.6 | 3.1 | 3.3 | 5.5 | Crystal form C |
| Crystal form N | FaSSIF | 6.5 | 20.1 | 35.8 | 38.7 | 36.5 | 6.4 | Crystal form N |
| | FeSSIF | 5.0 | 20.2 | 111.9 | 114.2 | 107.6 | 5.0 | Crystal form N |
| | FaSSGF | 1.6 | 20.0 | 13.6 | 18.7 | 16.1 | 1.6 | Crystal form N |
| | Water | - | 19.8 | 15.1 | 11.8 | 3.1 | 5.2 | Crystal form C |

### Performance Example 3 Stability studies of 7,8-dihydroxyflavone crystal form P tablets

The stability of the 7,8-dihydroxyflavonoid crystal form P tablets was studied under high temperature (60°C), high humidity (25°C/92.5% RH), illumination (25°C/4500 Lux), and acceleration (40°C/75% RH) conditions, and samples were taken for XRPD characterization at the third month and the sixth month, respectively. The results are shown in Table 6.

**Table 6**

| **Time interval** | **Condition** | **Crystal form** |
|---|---|---|
| 0 | High temperature 60°C | P |
| | High humidity 25°C/ 92.5% RH | P |
| | Illumination 25°C/ 4500 Lux | P |
| | Acceleration 40°C/75% RH | P |
| 3 Months | High temperature 60°C | P |
| | High humidity 25°C/ 92.5% RH | P |
| | Illumination 25°C/ 4500 Lux | P |
| | Acceleration 40°C/75% RH | P |
| 6 Months | High temperature 60°C | P |
| | High humidity 25°C/ 92.5% RH | P |
| | Illumination 25°C/ 4500 Lux | P |
| | Acceleration 40°C/75% RH | P |

### Performance Example 4 Stability studies of 7,8-dihydroxyflavone crystal form C tablets

The stability of the 7,8-dihydroxyflavonoid crystal form C tablets was studied under high temperature (60°C), high humidity (25°C/92.5% RH), illumination (25°C/4500 Lux), and acceleration (40°C/75% RH) conditions, and samples were taken for XRPD characterization at the third month and the sixth month, respectively. The results are shown in Table 7.

**Table 7**

| **Time interval** | **Condition** | **Crystal form** |
|---|---|---|
| 0 | High temperature 60°C | C |
| | High humidity 25°C/ 92.5% RH | C |
| | Illumination 25°C/ 4500 Lux | C |
| | Acceleration 40°C/75% RH | C |
| 3 Months | High temperature 60°C | C |
| | High humidity 25°C/ 92.5% RH | C |
| | Illumination 25°C/ 4500 Lux | C |
| | Acceleration 40°C/75% RH | C |
| 6 Months | High temperature 60°C | C |
| | High humidity 25°C/ 92.5% RH | C |
| | Illumination 25°C/ 4500 Lux | C |
| | Acceleration 40°C/75% RH | C |

### Performance Example 5 Dissolution studies of 7,8-dihydroxyflavone crystal form P tablets

The dissolution studies of 7,8-dihydroxyflavone crystal form P tablets were performed at different pH conditions. The results are shown in Table 8.

**Table 8**

| Time (minutes) | pH = 4.5 Medium | pH = 6.8 Medium |
|---|---|---|
| 15 | 67.9% | 68.2% |
| 30 | 81.5% | 82.0% |
| 45 | 87.4% | 85.6% |
| 60 | 89.9% | 90.7% |
| 90 | 93.7% | 92.5% |
| 120 | 94.7% | 96.6% |

### Performance Example 6 Dissolution studies of 7,8-dihydroxyflavone crystal form C tablets

The dissolution studies of 7,8-dihydroxyflavone crystal form C tablets were performed at different pH conditions. The results are shown in Table 9.

**Table 9**

| Time (minutes) | pH = 4.5 Medium | pH = 6.8 Medium |
|---|---|---|
| 15 | 47.7% | 47.5% |
| 30 | 55.1% | 58.2% |
| 45 | 58.8% | 64.1% |
| 60 | 60.8% | 67.6% |
| 90 | 63.6% | 71.6% |
| 120 | 64.8% | 73.6% |

### Performance Example 7 Dissolution studies of 7,8-dihydroxyflavone crystal form A tablets

The dissolution studies of 7,8-dihydroxyflavone crystal form A tablets were performed at different pH conditions. The results are shown in Table 10.

**Table 10**

| Time (minutes) | pH = 4.5 Medium | pH = 6.8 Medium |
|---|---|---|
| 15 | 33.9% | 33.2% |
| 30 | 58.8% | 57.9% |
| 45 | 68.9% | 68.2% |
| 60 | 76.1% | 75.1% |
| 90 | 84.2% | 82.7% |
| 120 | 89.6% | 89.0% |

In summary, the present disclosure provides a polymorph of 7,8-dihydroxyflavone and a preparation method therefor. The polymorph of 7,8-dihydroxyflavone provided by the present disclosure has more selectable pharmacoeconomic value.

## Claims

1. A crystal form P of 7,8-dihydroxyflavone, which has an X-ray powder diffraction pattern comprising characteristic peaks at about 8.3 ± 0.2°, 25.0 ± 0.2°, and 25.9 ± 0.2° expressed in 2θ angles.

2. The crystal form P of 7,8-dihydroxyflavone according to claim 1, wherein the X-ray powder diffraction pattern further comprises characteristic peaks at about 27.4 ± 0.2° and/or 31.4 ± 0.2°.

3. A crystal form C of 7,8-dihydroxyflavone, which has an X-ray powder diffraction pattern comprising characteristic peaks at about 11.1 ± 0.2°, 15.6 ± 0.2°, 27.0 ± 0.2°, and 28.3 ± 0.2° expressed in 2θ angles.

4. The crystal form C of 7,8-dihydroxyflavone according to claim 3, wherein the X-ray powder diffraction pattern further comprises characteristic peaks at about 5.6 ± 0.2°, 9.5 ± 0.2°, 15.5 ± 0.2°, 21.0 ± 0.2°, 24.3 ± 0.2° and/or 25.5 ± 0.2°.

5. The crystal form C of 7,8-dihydroxyflavone according to any one of claims 3 to 4, which is a hydrate, for example, a monohydrate.

6. A crystal form N of 7,8-dihydroxyflavone, which has an X-ray powder diffraction pattern comprising characteristic peaks at about 9.3 ± 0.2°, 23.8 ± 0.2°, 24.6 ± 0.2°, and 26.6 ± 0.2° expressed in 2θ angles.

7. The crystal form N of 7,8-dihydroxyflavone according to claim 6, wherein the X-ray powder diffraction pattern further comprises characteristic peaks at about 12.3 ± 0.2° and/or 21.1 ± 0.2°.

8. The crystal form N of 7,8-dihydroxyflavone according to any one of claims 6 to 7, which is a hydrate, for example, a dihydrate.

9. A pharmaceutical composition, which comprises (1) the crystal form P of 7,8-dihydroxyflavone according to any one of claims 1 to 2, the crystal form C of 7,8-dihydroxyflavone according to any one of claims 3 to 5, and/or the crystal form N of 7,8-dihydroxyflavone according to any one of claims 6 to 8, and (2) at least one pharmaceutically acceptable excipient.

10. A method for treating a disease associated with the aberrant TrkB signaling pathway such as central nerve injury, peripheral nerve injury, neurodegenerative diseases, psychiatric diseases, hereditary neurological disorders, ophthalmological diseases, metabolic diseases, pain, cardiovascular diseases, tumor or other related diseases, comprising administering to a subject (1) the crystal form P of 7,8-dihydroxyflavone according to any one of claims 1 to 2, the crystal form C of 7,8-dihydroxyflavone according to any one of claims 3 to 5, and/or the crystal form N of 7,8-dihydroxyflavone according to any one of claims 6 to 8, and (2) at least one pharmaceutically acceptable excipient.
